# EUROPEAN PATENT SPECIFICATION

(11) **EP 1 833 477 B1**
(45) Date of publication and mention of the grant of the patent: **02.06.2010**
(21) Application number: 05822740.6
(22) Date of filing: 14.12.2005
(51) Int. Cl.: A61K 31/4162, C07D 471/04, C07D 487/04, A61K 31/4985, A61K 31/519, A61P 29/00

(54) **PYRAZOLO-HETEROARYL COMPOUNDS USEFUL TO TREAT TNF-ALPHA AND IL-1 MEDIATED DISEASES**
PYRAZOLO-HETEROARYL-VERBINDUNGEN ZUR BEHANDLUNG TNF-ALPHA- UND IL-1-VERMITTELTER KRANKHEITEN
COMPOSÉS DE TYPE PYRAZOLO-HÉTÉROARYLES POUVANT ÊTRE EMPLOYÉS DANS LE TRAITEMENT DE MALADIES DUES À TNF-ALPHA ET IL-1

(30) Priority: 16.12.2004 GB 0427604
(43) Date of publication of application: 19.09.2007
(73) Proprietor: Novartis AG, 4056 Basel (CH); Novartis Pharma GmbH, 1230 Wien (AT)
(72) Inventor: RÉVÉSZ, Lászlo, CH-4106 Therwil (CH)
(74) Representative: Patent- und Rechtsanwaltssozietät Maucher, Börjes & Kollegen
(86) International application number: PCT/EP2005/013453
(87) International publication number: WO 2006/063820

(56) References cited:
- WO-A-03/029209
- WO-A-03/099820
- WO-A-2004/076450
- WO-A-2005/085248
- US-A1- 2003 207 900

## Description

This invention relates to pyrazolo-heteroaryl compounds, in particular to pyrazolo[3,4-.b.]pyridine, pyrazolo[3,4-.d.]pyrimidine and pyrazolo[3,4-.b.]pyrazine derivatives and to their use for preparing medicaments for treating TNFα and IL-1 mediate diseases such as rheumatoid arthritis and diseases of bone metabolism, e.g. osteoporosis.

Accordingly the present invention provides a compound of formula I 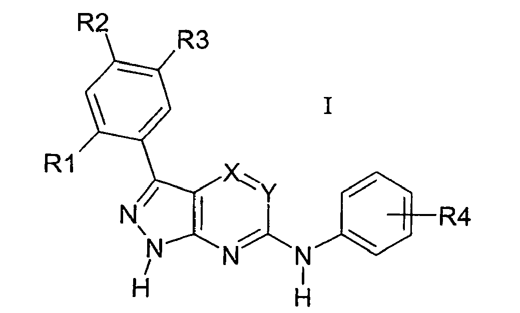 wherein
X and Y are independently carbon or nitrogen,
R1 is H, halogen, hydroxy, lower alkoxy, lower alkyl or halo-substituted lower alkyl;
R2 is H, or optionally substituted (heterocyclyl, lower alkyl, lower alkenyl, lower alkynyl or lower alkoxy);
R3 is H, or optionally substituted (heterocyclyl, lower alkyl, lower alkenyl, lower alkynyl or lower alkoxy); or
R2 and R3 are linked together to form a 4- to 6-membered heterocyclic ring containing one or more hetero atoms selected from O, S, N or NR, where R is H or lower alkyl;
R4 represents one, two or three halogen substituents which may be the same or different,
or a pharmaceutically-acceptable and -cleavable ester or acid addition salt thereof with the exception of [3-(2-chlorophenyl)-1H-pyrazolo[3,4-d]pyrimidin-6-yl]-(2-fluorophenyl]-amine, [3-(2-chlorophenyl)-1H-pyrazolo[3,4-b]pyrazin-6-yl]-(2,4-difluorophenyl)-amine and [3-(2-chlorophenyl)-1H-pyrazolo[3,4-d]pyrimidin-6-yl]-(2,4-difluorophenyl)-amine.

Preferably -X=Y- in formula I is -CH=N- or -N=CH- and the compounds of formula I are pyrazolo[3,4-.b.]pyridine, pyrazolo[3,4-.d.]pyrimidine and pyrazolo[3,4-.b.]pyrazine derivatives of formulae I', I", I"' 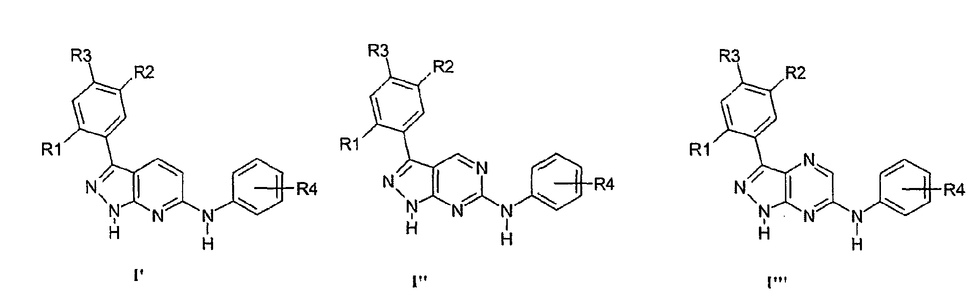 wherein the substituents R1, R2, R3 and R4 are as defined above with the exception of [3-(2-chlorophenyl)-1H-pyrazolo[3,4-d]pyrimidin-6-yl]-(2-fluorophenyl]-amine, [3-(2-chlorophenyl)-1H-pyrazolo[3,4-b]pyrazin-6-yl]-(2,4-difluorophenyl)-amine and [3-(2-chlorophenyl)-1H-pyrazolo[3,4-d]pyrimidin-6-yl]-(2,4-difluorophenyl)-amine.

Above and elsewhere in the present description the following terms have the following meanings.

Halo or halogen denote I, Br, Cl or F, preferably Cl or F.

The term "lower" referred to above and hereinafter in connection with organic radicals or compounds respectively defines such as branched or unbranched with up to and including 7, preferably up to and including 4 and advantageously one or two carbon atoms.

A lower alkyl group is branched or unbranched and contains 1 to 7 carbon atoms, preferably 1-4 carbon atoms. Lower alkyl represents for example methyl, ethyl, propyl, butyl, isopropyl or isobutyl.

Halo-substituted lower alkyl is C₁-C₇lower alkyl substituted by up to 6 halo atoms.

A lower alkoxy group is branched or unbranched and contains 1 to 7 carbon atoms, preferably 1-4 carbon atoms. Lower alkoxy represents for example methoxy, ethoxy, propoxy, butoxy, isopropoxy, isobutoxy or tertiary butoxy.

A lower alkene or alkenyl group is branched or unbranched and contains 2 to 7 carbon atoms, preferably 1-4 carbon atoms and contains at least one carbon-carbon double bond. Lower alkene or lower alkenyl represents for example vinyl, propenyl, butenyl, isopropenyl or isobutenyl.

A lower alkyne or alkynyl group is branched or unbranched and contains 2 to 7 carbon atoms, preferably 1-4 carbon atoms and contains at least one carbon-carbon triple bond. Lower alkyne or alkynyl represents for example ethynyl, propynyl, butynyl, isopropynyl or isobutynyl.

Heterocyclyl may be aromatic, i.e. as C₃₋₁₈heteroaryl, partially unsaturated comprising from 3 to 18 ring members, or saturated, i.e. as C₃₋₁₈heterocycloalkyl, and comprises at least 3 ring atoms, at least one of which is a hetero atom, e.g. O, S, N, or NR, where R is H or lower alkyl.

R1, R2 and R3 may be further substituted by one or more, e.g. up to six, substituents independently selected from halo, OH, CN, lower alkyl, lower alkoxy, heterocyclyl or NR5R6 where R5 and R6 are independently H or lower alkyl. The lower alkyl, lower alkoxy, heterocyclyl or NR5R6 substituents on R1, R2 and R3 may be further substituted by one or more, each by up to six, more usually up to three, e.g. 1 or 2, substituents independently selected from halo, OH, CN, lower alkyl, lower alkoxy, heterocyclyl or NR5R6, where R5 and R6 are as defined above.

R1 is preferably halo, C₁-C₄ lower alkyl, C₁-C₄lower alkoxy or halo-substituted C₁-C₄ lower alkyl. Most preferably R1 is Cl, F, methoxy or CF₃.

R2 and R3 are preferably, independently of one another, H or optionally substituted (C₁-C₄ lower alkyl, C₁-C₄ lower alkoxy, C₁-C₇alkenyl, C₁-C₇alkynyl, C₅-C₇N-heterocyclyl, C₅-C₇heterocyclylC₁-C₄lower alkoxy, C₅-C₇heterocyclylC₁-C₄lower alkyl, C₅-C₇heterocyclylC₁-C₄lower alkenyl, C₅-C₇heterocyclylC₁-C₄lower alkynyl) wherein the C₅-C₇heterocyclyl optionally contains a second hetero atom, e.g. O, S, N, NR (where R is H or lower alkyl), and the optional substitution comprises 1 or 2 substituents, separately selected from C₁-C₄ alkyl, halogen, hydroxy, C₁-C₄ alkoxy, or optionally mono-or di-N-C₁₋₄alkyl substituted amino.

In particular R2 is H, C₁-C₄ lower alkoxy, or C₅-C₇heterocyclylC₁-C₄lower alkoxy. For example, in particular embodiments R2 is H, methoxy and N-morpholinylethoxy.

More preferably R3 is H or optionally substituted (C₁-C₄ lower alkyl, C₁-C₄ lower alkenyl, C₁-C₄ lower alkynyl, C₁-C₄ lower alkoxy, morpholino, pyridyl, morpholino-C₁-C₄ lower alkoxy, imidazolyl, piperidyl, C₁-C₄ lower alkylamino-C₁-C₄ lower alkyL, piperazyl C₁-C₄ lower alkyl, morpholino C₁-C₄ lower alkyl, tetrahydropyranylamino C₁-C₄ lower alkyl, pyridylaminocarbonyl, morpholino C₁-C₄ lower alkenyl, piperidyl C₁-C₄ lower alkenyl, piperazyl C₁-C₄ lower alkenyl, morpholino C₁-C₄ lower alkynyl, piperidyl C₁-C₄ lower alkynyl, piperazyl C₁-C₄ lower alkynyl).

Particular examples of substituents as R3 include: H, methoxy, N-morpholino, pyrid-4-yl, pyrid-2-ylaminocarbonyl2-(N-morpholino)ethoxy, 1,5-dimethylimidazol-3-yl, 1-methyl-4-hydroxypiperid-4-yl, methylaminomethyl, 1-methylpiperaz-4-ylmethyl, piperaz-1-ylmethyl, propylaminomethyl, tetrahydropyran-4-ylaminomethyl, N-morpholinylmethyl, methylaminomethyl, methoxy, 3-amino-3-methyl-but-1-ynyl, 3-amino-3-methyl-butyl, 3-hydroxy-3-methyl-but-1-ynyl, 3-hydroxy-3-methyl-butyl, 3-dimethylaminoprop-1-ynyl, 3-morpholin-4-yl-prop-1-ynyl, 3-(4-methyl-piperazin-1-yl)-prop-1-ynyl, 1-methyl-4-hydroxy-piperid-4-ylethynyl, (E)-3-dimethylamino-propenyl, (E)-3-morpholin-4-ylpropenyl, (E)-3-(4-methyl-piperazi-1-yl)-propenyl, wherein the optional substituents are as defined above.

When R2 and R3 are linked to form a heterocylic ring they are preferably linked by a methylenedioxy linker or the heterocyclic ring is preferably an imidazole ring optionally substituted by 1 or 2 substituents, separately selected from C₁₋₄alkyl, halogen, hydroxy, C₁₋₄alkoxy, or optionally mono-or di-N-C₁₋₄alkyl substituted amino. Most preferably when R2 and R3 are linked to form a heterocylic ring they are preferably linked by a methylenedioxy linker.

A preferred significance for R4 is a dihalo, more preferably difluoro, especially 2,4-difluoro.

Thus in preferred embodiments the invention provides compounds of Formulae II 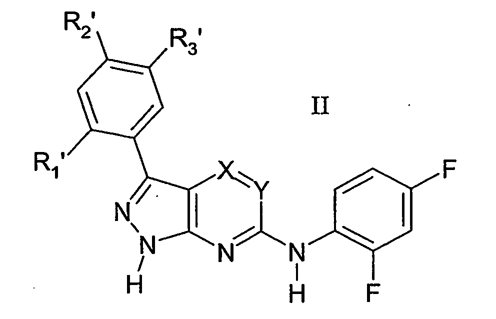 Wherein
X and Y are independently carbon or nitrogen,
R1' is halo, C₁-C₄ lower alkyl, C₁-C₄lower alkoxy or halo-substituted C₁-C₄ lower alkyl;
R2' and R3' are, independently of one another, H or optionally substituted (C₁-C₄ lower alkyl, C₁-C₄ lower alkoxy, C₁-C₇alkenyl, C₁-C₇alkynyl, C₅-C₇N-heterocyclyl, C₅-C₇heterocyclylC₁-C₄lower alkoxy, C₅-C₇heterocyclylC₁-C₄lower alkyl, C₅-C₇heterocyclylC₁-C₄lower alkenyl, C₅-C₇heterocyclylC₁-C₄lower alkynyl) wherein the C₅-C₇heterocyclyl optionally contains a second hetero atom and the optional substitution comprises 1 or 2 substituents, separately selected from C₁-C₄ alkyl, halogen, hydroxy, C₁-C₄ alkoxy, or optionally mono-or di-N-C₁₋₄alkyl substituted amino or
R2' and R3' are linked by a methylenedioxy linker or are linked in an imidazole ring optionally substituted by 1 or 2 substituents, separately selected from C₁₋₄alkyl, halogen, hydroxy, C₁₋₄alkoxy, or optionally mono-or di-N-C₁₋₄alkyl substituted amino;
or a pharmaceutically-acceptable and -cleavable ester or acid addition salt thereof with the exception of [3-(2-chlorophenyl)-1H-pyrazolo[3,4-b]pyrazin-6-yl]-(2,4-difluorophenyl)-amine and [3-(2-chlorophenyl)-1H-pyrazolo[3,4-d]pyrimidin-6-yl]-(2,4-difluorophenyl)-amine.

In particular the invention includes the following compounds:
Example 1: (2,4-Difluoro-phenyl)-[3-(2-methoxy-phenyl)-1.H.-pyrazolo[3,4-.b.]pyridin-6-yl]-amine
Example 2: [3-(6-Chloro-benzo[1,3]dioxol-5-yl)-1.H.-pyrazolo[3,4-.b.]pyridin-6-yl]-(2,4-difluoro-phenyl)-amine
Example 3: [3-(2-Chloro-phenyl)-1.H.-pyrazolo[3,4-.b.]pyridin-6-yl]-(2,4-difluoro-phenyl)-amine
Example 4: (2,4-Difluoro-phenyl)-[3-(2-trifluoromethyl-phenyl)-1.H.-pyrazolo[3,4-.b.]pyridin-6-yl]-amine
Example 5: (2,4-Difluoro-phenyl)-[3-(2,4-dimethoxy-phenyl)-1.H.-pyrazolo[3,4-.b.]pyridin-6-yl]-amine
Example 6: [3-(2-Chloro-4,5-dimethoxy-phenyl)-1.H.-pyrazolo[3,4-.b.]pyridin-6-yl]-(2,4-difluoro-phenyl)-amine
Example 7: (2,4-Difluoro-phenyl)-[3-(2-methoxy-5-morpholin-4-yl-phenyl)-1.H.-pyrazolo[3,4-.b.]pyridin-6-yl]-amine
Example 22: (2,4-Difluoro-phenyl)-{3-[2-methoxy-5-(4-methyl-piperazin-1-yl)-phenyl]-1.H.-pyrazolo[3,4-.b.]pyridin-6-yl}-amine
Example 8: (2,4-Difluoro-phenyl)-[3-(2-methoxy-5-pyridin-4-yl-phenyl)-I.H.-pyrazolo[3,4-.b.]pyridin-6-yl]-amine
Example 13: (2,4-Difluoro-phenyl)-[3-(2-methoxy-5-methylaminomethyl-phenyl)-1.H.-pyrazolo[3,4-.b.]pyridin-6-yl]-amine
Example 14: (2,4-Difluoro-phenyl)-{3-[2-methoxy-5-(4-methyl-piperazin-1-ylmethyl)-phenyl]-1.H.-pyrazolo[3,4-.b.]pyridin-6-yl}-amine
Example 12: 4-{3-[6-(2,4-Difluoro-phenylamino)-1.H.-pyrazolo[3,4-.b.]pyridin-3-yl]-4-methoxy-phenyl}-1-methyl-piperidin-4-ol
Example 15: (2,4-Difluoro-phenyl)-[3-(2-methoxy-5-morpholin-4-ylmethyl-phenyl)-1.H.-pyrazolo[3,4-.b.]pyridin-6-yl]-amine
Example 16: (2,4-Difluoro-phenyl)-[3-(2-methoxy-5-piperazin-1-ylmethyl-phenyl)-1.H.-pyrazolo[3,4-.b.]pyridin-6-yl]-amine
Example 20: {3-[5-(3-Amino-3-methyl-but-1-ynyl)-2-methoxy-phenyl]-1.H.-pyrazolo[3,4-.b.]pyridin-6-yl}-(2,4-difluoro-phenyl)-amine
Example 21: {3-[5-(3-Amino-3-methyl-butyl)-2-methoxy-phenyl]-1.H.-pyrazolo[3,4-.b.]pyridin-6-yl}-(2,4-difluoro-phenyl)-amine
Example 9: {3-[2-Chloro-5-methoxy-4-(2-morpholin-4-yl-ethoxy)-phenyl]-1.H.-pyrazolo[3,4-.b.]pyridin-6-yl}-(2,4-difluoro-phenyl)-amine
Example 10: {3-[2-Chloro-4-methoxy-5-(2-morpholin-4-yl-ethoxy)-phenyl]-1.H.-pyrazolo[3,4-.b.]pyridin-6-yl}-(2,4-difluoro-phenyl)-amine
Example 17: (2,4-Difluoro-phenyl)-[3-(2-methoxy-5-propylaminomethyl-phenyl)-1.H.-pyrazolo[3,4-.b.]pyridin-6-yl]-amine
Example 18: (2,4-Difluoro-phenyl)-(3-{2-methoxy-5-[(tetrahydro-pyran-4-ylamino)-methyl]-phenyl}-1.H.-pyrazolo[3,4-.b.]pyridin-6-yl)-amine
Examples 11 and 19: (2,4-Difluoro-phenyl)-{3-[5-(1,2-dimethyl-1.H.-imidazol-4-yl)-2-methoxy-phenyl]-1.H.-pyrazolo[3,4-.b.]pyridin-6-yl}-amine
Example 23: 3-[6-(2,4-Difluoro-phenylamino)-1.H.-pyrazolo[3,4-.b.]pyridin-3-yl]-4-methoxy-.N.-pyridin-2-yl-benzamide
Example 24: {3-[5-(3-Amino-3-methyl-but-1-ynyl)-2-methoxy-phenyl]-1.H.-pyrazolo[3,4-.d.]pyrimidin-6-yl}-(2,4-difluoro-phenyl)-amine
Example 25: 4-{3-[6-(2,4-Difluoro-phenylamino)-1.H.-pyrazolo[3,4-.d.]pyrimidin-3-yl]-4-methoxy-phenyl}-2-methyl-but-3-yn-2-ol
Example 26: 4-{3-[6-(2,4-Difluoro-phenylamino)-1.H.-pyrazolo[3,4-.d.]pyrimidin-3-yl]-4-methoxy-phenyl}-2-methyl-butan-2-ol
Example 27: (2,4-Difluoro-phenyl)-{3-[5-(3-dimethylamino-prop-1-ynyl)-2-methoxy-phenyl]-1.H.-pyrazolo[3,4-.d.]pyrimidin-6-yl}-amine
Example 28: (2,4-Difluoro-phenyl)-{3-[2-methoxy-5-(3-morpholin-4-yl-prop-1-ynyl)-phenyl]-1.H.-pyrazolo[3,4-.d.]pyrimidin-6-yl}-amine
Example 29: (2,4-Difluoro-phenyl)-(3-{2-methoxy-5-[3-(4-methyl-piperazin-1-yl)-prop-1-ynyl]-phenyl}-1.H.-pyrazolo[3,4-.d.]pyrimidin-6-yl)-amine
Example 30: 4-{3-[6-(2,4-Difluoro-phenylamino)-1.H.-pyrazolo[3,4-.d.]pyrimidin-3-yl]-4-methoxy-phenylethynyl}-1-methyl-piperidin-4-ol
Example 31: (2,4-Difluoro-phenyl)-{3-[5-((E)-3-dimethylamino-propenyl)-2-methoxy-phenyl]-1.H.-pyrazolo[3,4-.d.]pyrimidin-6-yl}-amine
Example 32: (2,4-Difluoro-phenyl)-{3-[2-methoxy-5-((E)-3-morpholin-4-yl-propenyl)-phenyl]-1.H.-pyrazolo[3,4-.d.]pyrimidin-6-yl}-amine
Example 33: (2,4-Difluoro-phenyl)-(3-{2-methoxy-5-[(E)-3-(4-methyl-piperazin-1-yl)-propenyl]-phenyl}-1.H.-pyrazolo[3,4-.d.]pyrimidin-6-yl)-amine
Example 34: 4-{3-[6-(2,4-Difluoro-phenylamino)-1.H.-pyrazolo[3,4-.b.]pyrazin-3-yl]-4-methoxy-phenyl}-2-methyl-but-3-yn-2-ol
Example 35: (2,4-Difluoro-phenyl)-{3-[5-(3-dimethylamino-prop-1-ynyl)-2-methoxy-phenyl]-1.H.-pyrazolo[3,4-.b.]pyrazin-6-yl}-amine
   or a pharmaceutically-acceptable and -cleavable ester or acid addition salt thereof.

The novel pyrazolopyridine, pyrazolopyrimidine and pyrazolopyrazine compounds of the invention, in particular the compounds of formulae I and II and the specific compounds listed above are hereinafter referred to "Agents of the Invention".

The Agents of the Invention which comprise free hydroxyl groups may also exist in the form of pharmaceutically acceptable, physiologically cleavable esters, and as such are included within the scope of the invention. Such pharmaceutically acceptable esters are preferably prodrug ester derivatives, such being convertible by solvolysis or cleavage under physiological conditions to the corresponding Agents of the Invention which comprise free hydroxyl groups. Suitable pharmaceutically acceptable prodrug esters are those derived from a carboxylic acid, a carbonic acid monoester or a carbamic acid, advantageously esters derived from an optionally substituted lower alkanoic acid or an arylcarboxylic acid.

Agents of the Invention may also exist in the form of pharmaceutically acceptable salts, and as such are included within the scope of the invention. Pharmaceutically acceptable salts include acid addition salts with conventional acids, for example, mineral acids, e.g., hydrochloric acid, sulfuric or phosphoric acid, or organic acids, for example, aliphatic or aromatic carboxylic or sulfonic acids, e.g., acetic, propionic, succinic, glycolic, lactic, malic, tartaric, citric, ascorbic, maleic, fumaric, hydroxymaleic, pyruvic, pamoic, methanesulfonic, toluenesulfonic, naphthalenesulfonic, sulfanilic or cyclohexylsulfamic acid; also amino acids, such as arginine and lysine. For compounds of the invention having acidic groups, for example, a free carboxy group, pharmaceutically acceptable salts also represent metal or ammonium salts, such as alkali metal or alkaline earth metal salts, e.g., sodium, potassium, magnesium or calcium salts, as well as ammonium salts, which are formed with ammonia or suitable organic amines.

Agents of the Invention of formula II' 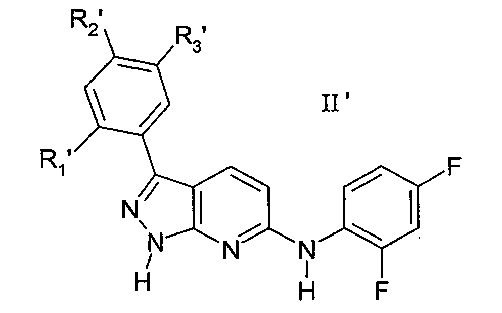 wherein R1', R2', and R3' are as defined above may be prepared by coupling a 6-chloro-3-phenyl-1.H.-pyrazolo[3,4-.b.]pyridine of formula III 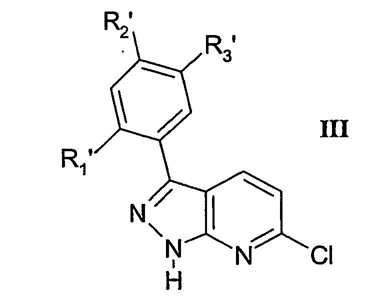 wherein R1', R2' and R3' are as defined above, with 2,4-difluoroaniline. Preferably the coupling reaction is carried out as a Buchwald condensation; for instance, in solution, e.g. in hot dioxane, in the presence of e.g. R-(+)-BINAP, Pd(OAc)₂, and NaOtBu with refluxing e.g. for about 3 hours.

The compound of formula III may be obtained by cyclisation of the corresponding (2,6-dichloro-pyridin-3-yl)-phenyl-methanone of formula IV 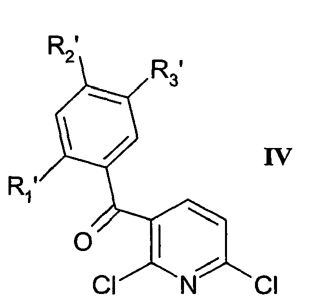 wherein R1', R2' and R3' are as defined above, with hydrazine (H₂NNH₂); for instance, in organic solvent, e.g. EtOH/1-BuOH, under reflux.

Alternatively the compound of formula III in which R2' is H may be obtained by coupling a bromo derivative of formula V 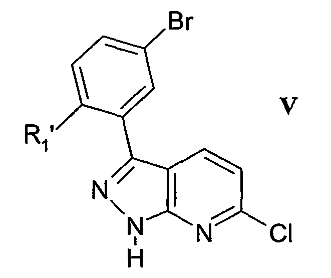 wherein R1' is as defined above, with a corresponding R3' precursor, e.g. N-methyl-4-piperidone when R3' is 1-hydroxy-4-N-methylpiperidine-1-yl, N-methylpiperazine when R3' is 4-N-methylpiperazin-1-yl, or 3-amino-3-methylbutyn-1-yl when R3' is 3-amino-3-methylbutyn-1-yl. Conditions used for these coupling reactions are described hereinafter in the Examples.

Compounds of formula II in which R3' is an alkyl substituent, e.g. amino substituted alkyl, such as a 3-amino-3-methylbutyn-1-yl substituent, may be prepared by reduction of the corresponding alkyne substituted, e.g. 3-amino-3-methylbutyn-1-yl, compound of formula II; for instance as hereinafter described in the Examples.

Compounds of formula III in which R2' is H and R3' is -CH₂-NR5R6, wherein R5 is H and R6 is lower alkyl or R5 and R6 are linked together in an N-heterocyclyl comprising at least 3 ring atoms and optionally a further heteroatom selected from O, S, N or NR where R is H or lower alkyl, may be prepared by reaction of a 3-(6-chloro-1.H.-pyrazolo[3,4-.b.]pyridin-3-yl)-4-methoxy-benzaldehyde compound of formula VI 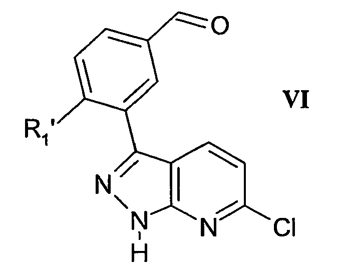 wherein R1' is as defined above, with a lower alkyl amine or N-heterocyclic precursor; for instance as hereinafter described in the Examples. Compounds of formula III in which R2' is H and R3' is -CH₂-NR5R6, wherein R5 is H and R6 is lower alkyl or R5 and R6 are linked together in an N-heterocyclyl comprising at least 3 ring atoms and optionally a further heteroatom selected from O, S, N or NR where R is H or lower alkyl, may be converted to compounds of formula II as described above.

Compounds of formula VI may be prepared by reacting a bromo derivative of formula V with DMF; for instance after treatment with an alkyl lithium agent such as nBuLi, preferably with cooling.

Compounds of formula IV as defined above may be obtained by oxidation of the corresponding alcohol of formula VII 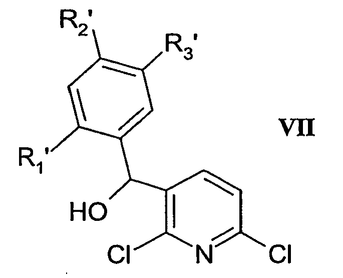 wherein R1', R2' and R3' are as defined above. Oxidation may be carried out using Jones reagent; for instance as hereinafter described in the Examples.

The alcohol of formula VII may be obtained by coupling of 2,6 dichloropyridine with the appropriate benzaldehyde precursor of formula VIII 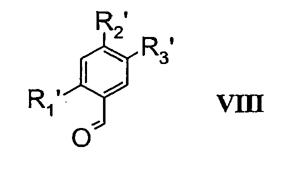 wherein R1', R2' and R3' are as defined above. For example, the coupling reaction may be carried out in solution, e.g. in THF, preferably containing diisopropylamine which has been pretreated with nBuLi, preferably with cooling.

Novel synthetic steps as described above, in particular the coupling of compounds of formula III with 2,6-difluoroaniline, are included within the scope of the present invention.

Accordingly the invention further provides a process for the preparation of Agents of the Invention of formula II 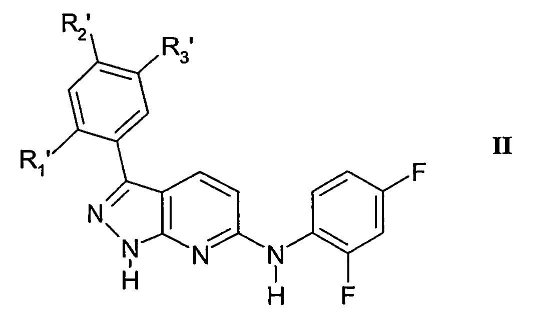 wherein R1', R2', and R3' are as defined above, comprising coupling a 6-chloro-3-phenyl-1.H.-pyrazolo[3,4-.b.]pyridine of formula III 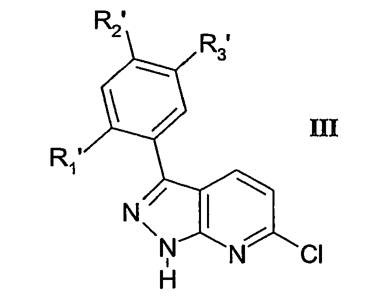 wherein R1', R3' and R3' are as defined above, with 2,4-difluoroaniline.

The invention is further described by way of illustration only in the following examples which relate to the preparation of Agents of the Invention of formula II.

### EXAMPLES

Methods are specifically described for preparation of Agents of the Invention. Agents of the Invention may be prepared according to scheme 1 from aldehydes I:

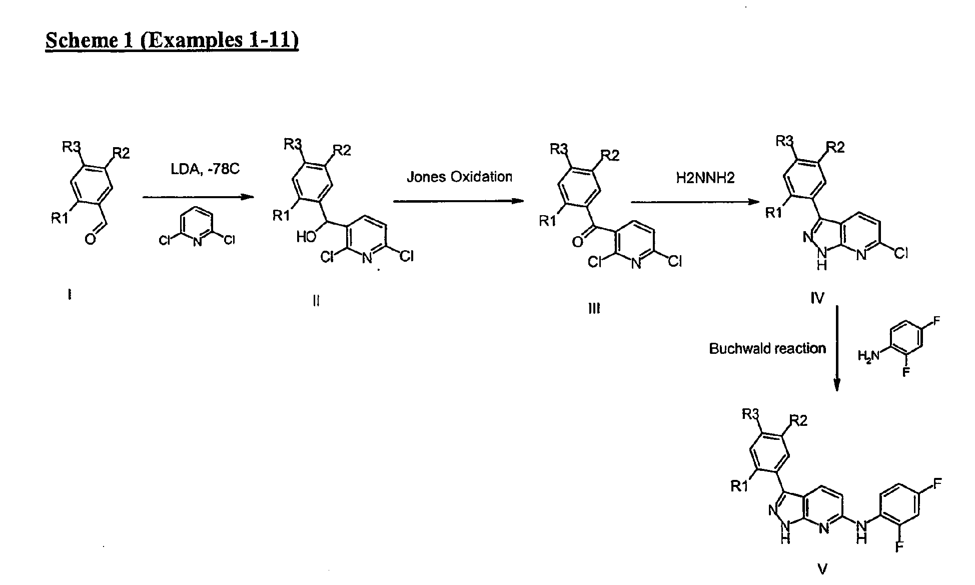

The preparation of (2,4-difluoro-phenyl)-[3-(2-methoxy-phenyl)-1.H.-pyrazolo[3,4-.b.]pyridin-6-yl]-amine (Example 1) exemplifies the 4-step synthetic method of scheme 1 from commercially available 2-methoxybenzaldehyde:

### Example 1: (2,4-Difluoro-phenyl)-[3-(2-methoxy-phenyl)-1.H.-pyrazolo[3,4-.b.]pyridin-6-yl]-amine

### 1 a) (2,6-Dichloro-pyridin-3-yl)-(2-methoxy-phenyl)-methanol

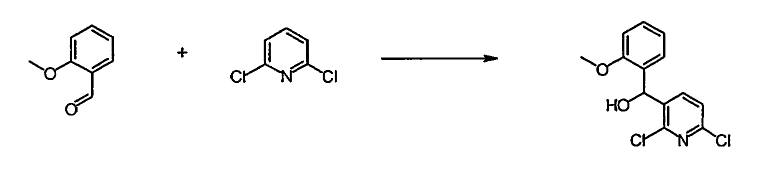

Diisopropylamine (10.3ml; 74mmol) in THF (200ml) is cooled to -78°C and treated with nBuLi (42ml; 67.6mmol of a 1.6M solution in hexane). After 5min at -78°C, 2,6-dichloropyridine (10g; 67.6 mmol) in THF (20ml) is added dropwise and stirred for 50min at -78°C. 2-Methoxybenzaldehyde (8.75g; 64.3mmol) in THF (15ml) is added dropwise. After 10min of stirring at -75C, the reaction mixture is warmed to -30C and poured on aqueous 20% NaCl-solution (500ml) and extracted with TBME three times. The combined organic phases are dried over Na₂SO_{4,} evaporated to dryness and the residue purified by chromatography (SiO₂; acetone/hexanes 5/95 > 3/7) to yield the title compound (16.0g; 87%) as a slightly colored viscuous oil.
1H-NMR (400MHz; DMSO): 3.73 (s, 3H): 6.07 (d, 1H); 6.15 (d, 1H); 6.97 (m, 2H); 7.29 (m, 2H); 7.55 (d, 1H); 7.83 (d, 1H).
MS (m/z) ES-: 284 (MH-; 30); 282 (35); 146 (100).

### 1b) (2,6-Dichloro-pyridin-3-yl)-(2-methoxy-phenyl)-methanone

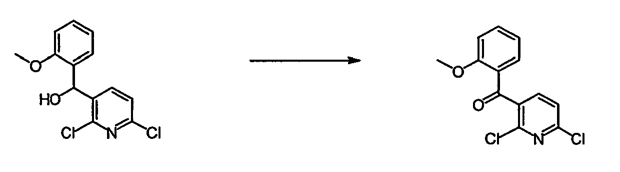

(2,6-Dichloro-pyridin-3-yl)-(2-methoxy-phenyl)-methanol (16g; 56.3mol) is dissolved in acetone (250ml ) and treated with Jones reagent (24ml; 56mmol) for 5min. The reaction mixture is diluted with hexanes (1000ml), filtered through a short bed of SiO₂, yielding the title compound (14.3g; 90%) after evaporation of the solvent as brownish crystals.
1H-NMR (400MHz; DMSO): 3.60 (s, 3H); 7.13 (t, 1H); 7.18 (d, 1H); 7.62-7.75 (m, 3H); 7.98 (d, 1H);
MS (m/z) EI: 283 (M+1, 50); 281 (M-1, 70); 246 (100); 135 (70); 77 (50).

### 1c) 6-Chloro-3-(2-methoxy-phenyl)-1.H.-pyrazolo[3,4-.b.]pyridine

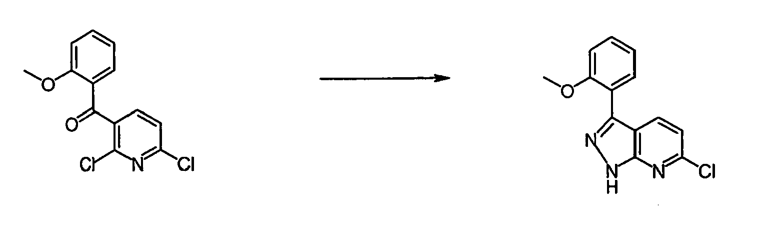

(2,6-Dichloro-pyridin-3-yl)-(2-methoxy-phenyl)-methanone (4g; 14.2mmol) dissolved in EtOH/1-BuOH (66ml 10/1) is treated with H₂NNH₂.H2O (24% in water)(5ml; 37.5mmol) under reflux for 30min. A second portion of H₂NNH₂ . H2O (24% in water)(4ml; 30mmol) is added and refluxed for another 30min. 1-BuOH is evaporated and the residual aqueous phase extracted with TBME three times. The combined organic phases are dried over Na₂SO₄, evaporated to dryness and the residue purified by chromatography (SiO₂; acetone/hexanes 2/8) to yield the title compound in pure form after triturating with a small volume of acetone (800mg; 22%) as yellow crystals.
1H-NMR (400MHz; DMSO): 3.82 (s, 3H); 7.09 (t, 1H); 7.22 (d, 1H); 7.27 (d, 1H); 7.47 (t, 1H); 7.64 (dd, 1H); 8.21 (d, 1H).
MS (m/z) EI: 259 (M+, 100); 230 (40); 194 (30); 152 (25); 77 (25).

### 1d): (2,4-Difluoro-phenyl)-[3-(2-methoxy-phenyl)-1.H.-pyrazolo[3,4-.b.]pyridin-6-yl]-amine

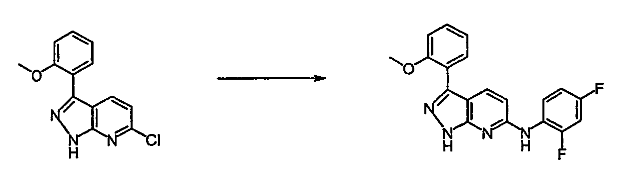

6-Chloro-3-(2-methoxy-phenyl)-1.H.-pyrazolo[3,4-.b.]pyridine (710mg; 2.74mmol) is dissolved in hot dioxane (10ml), R-(+)-BINAP (40mg; 0.06mmol), Pd(OAc)₂ (40mg; 0.17mmol), 2,4-difluoroaniline (1.1ml; 10.9mmol) and NaOtBu (1.05g; 10.9mmol) are sequentially added and the reaction mixture refluxed for 3 hours. It is then poured directly on a silica gel column and purified by chromatography (TBME/hexanes 4/6 > 8/2, then acetone/hexanes 1/1) to yield the title compound as off-white crystals after recrystallisation from acetone/hexanes (675mg; 69%).
1H-NMR (400MHz; DMSO): 3.85 (s, 3H); 6.81 (d, 1H); 7.07 (t, 1H); 7.12 (t, 1H); 7.20 (1H); 7.35 (t, 1H); 7.43 (t, 1H); 7.60 (d, 1H); 7.88 (d, 1H); 8.20 (m, 1H); 8.98 (s, 1H); 13.1 (s, 1H). MS (m/z) ES: 351 (MH-, 100); 331 (20).
The compounds of example 2, 3, 4 and 5 are prepared by analogy to example 1 from commercially available aldehydes:

### Example 2: [3-(6-Chloro-benzo[1,3]dioxol-5-yl)-1.H.-pyrazolo[3.4-.b.]pyridin-6-yl]-(2,4-difluoro-phenyl)-amine

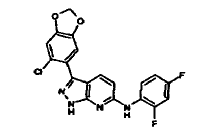

1H-NMR (400MHz; DMSO): 6.15 (s, 2H); 6.82 (d, 1H); 7.08 (s, 1H); 7.12 (dt, 1H); 7.23 (s, 1H); 7.32 (dt, 1H); 7.78 (d, 1H); 8.13 (m, 1H); 9.04 (s, 1H), 13.2 (s, 1H).
MS (m/z) Cm: 401 (MH+, 100); 259 (35); 213 (40); 179 (60); 169 (70); 141 (80); 131 (100).

### Example 3: [3-(2-Chloro-phenyl)-1.H.-pyrazolo[3,4-.b.]pyridin-6-yl]-(2,4-difluoro-phenyl)-amine

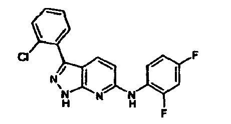

1H-NMR (400MHz; DMSO): 6.84 (d, 1H); 7.13 (bt, 1H); 7.33 (bt, 1H); 7.48 (m, 2H); 7.62 (m, 2H); 7.81 (d, 1H); 8.14 (m, 1H); 9.05 (s, 1H), 13.2 (s, 1H).
MS (m/z) ES: 355 (MH-, 100); 335 (15).

### Example 4: (2,4-Difluoro-phenyl)-[3-(2-trifluoromethyl-phenyl)-1.H.-pyrazolo[3,4-.b.]pyridin-6-yl]-amine

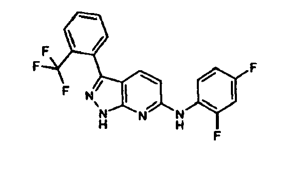

1H-NMR (400MHz; DMSO): 6.82 (d, 1H); 7.11 (bt, 1H); 7.33 (bt, 1H); 7.63 (d, 1H); 7.71 t, 2H); 7.80 (t, 1H); 7.92 (d, 1H); 8.12 (m, 1H); 9.05 (s, 1H), 13.2 (s, 1H).
MS (m/z) ES: 389 (MH-, 100); 369 (30).

### Example 5: (2,4-Difluoro-phenyl)-[3-(2,4-dimethoxy-phenyl)-1.H.-pyrazolo[3,4-.b.]pyridin-6-yl]-amine

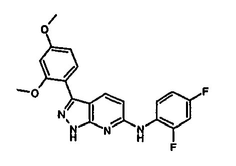

1H-NMR (400MHz; DMSO): 3.82 (s, 3H); 3.84 (s, 3H); 6.64 (dd, 1H); 6.72 (d, 1H); 6.78 (d, 1H); 7.10 (bt, 1H); 7.32 (bt, 1H); 7.50 (d, 1H); 7.83 (d, 1H); 8.18 (m, 1H); 8.94 (s, 1H); 13.2 (s, 1H).
MS (m/z) ES: 381 (MH-, 100); 361 (50).

Aldehydes **1-5** and **8** (scheme 2) are prepared as follows before being converted into the products of Example 6-10 according to scheme 1 by analogy to the experimental procedures described for Example 1. 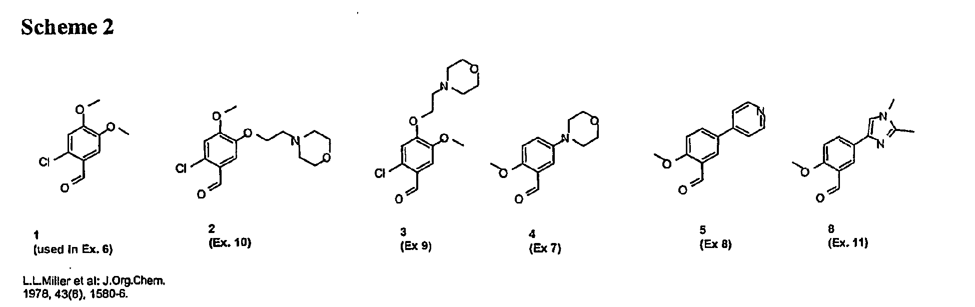 Preparation of aldehyde **1**: according to L.L.Miller et al J.Org.Chem 1978, 43(8), 1580-6.

### Preparation of 2-chloro-4-methoxy-5-(2-morpholin-4-yl-ethoxy)-benzaldehyde (2)

### a) 2-Chloro-4-methoxy-5-hydroxybenzaldehyde

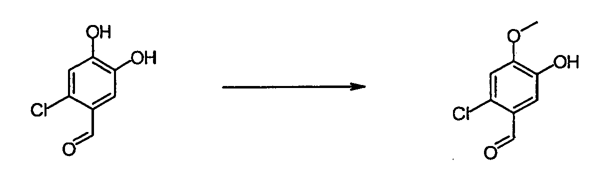

2-Chloro-4,5-dihydroxybenzaldehyde (Kaiser, C. et al in J.Org.Chem. 1974, 17(10), 1071-5)(5.7g; 33.1mmol), MeI (2.0ml; 33.1mmol) and K₂CO₃ (4.6g; 33.1mmol) are refluxed in acetone (250ml) for 1.5 hours. Acetone is evaporated and the residue taken up in TBME and extracted three times with TBME. The combined organic phases are dried over Na₂SO₄ and evaporated to dryness. Purification via chromatography (acetone/hexanes 15/85) yields in the first fraction 2-chloro-4,5-dimethoxybenzaldehyde (1.4g; 23%), followed by the title compound 2-chloro-4-methoxy-5-hydroxybenzaldehyde (2.45g; 40%; colorless crystals), 2-chloro-4-hydroxy-5-methoxybenzaldehyde (0.23g; 3.8%) and 2-chloro-4,5-dihydroxybenzaldehyde (2.11 g; 37% unreacted starting material).
1H-NMR (400MHz; CHCl₃): 3.90 (s, 3H); 5.52 (s, 1H); 6.81 (s, 1H); 7.37 (s, 1H); 10.22 (s, 1H).
MS (m/z) ES-: 187 (30); 185 (MH-, 100).

### b) 2-Chloro-4-methoxy-5-(2-morpholin-4-yl-ethoxy)-benzaldehyde (2)

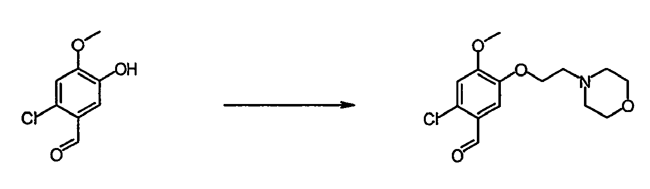

2-Chloro-4-methoxy-5-hydroxybenzaldehyde (500mg; 2.7mmol), N-(2-chloroethyl)morpholin.HCl (510mg; 2.7mmol) and K₂CO₃ (830mg; 6mmol) are refluxed in acetonitrile (3ml) for 3 hours. The solvent is evaporated, the residue taken up in acetone (100ml), filtered, concentrated until the title compound separated as slightly yellow crystals (487mg; 60%).
1H-NMR (400MHz; DMSO): 2.50 (bt, 4H); 2.72 (t, 2H); 3.58 (bt, 4H); 3.92 (s, 3H); 4.15 (t, 2H); 7.20 (s, 1H); 7.40 (s, 1H); 10.21 (s, 1H). Regiochemistry is proven by ROESY.
MS (m/z) EI: 299 (M+, 60); 100 (100).

### Preparation of 2-chloro-5-methoxy-4-(2-N-morpholinylethyloxy)benzaldehyde (3)

### a) 2-Chloro-5-hydroxy-4-(2-N-morpholinylethyloxy)benzaldehyde

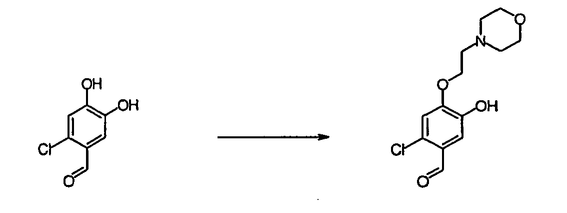

2-Chloro-4,5-dihydroxybenzaldehyde (Kaiser, C. et al in J.Org.Chem. 1974, 17(10), 1071-5)(500mg; 2.9mmol), N-(2-chloroethyl)morpholin.HCl (540mg; 2.9mmol), K₂CO₃ (880mg; 6.4mmol) are refluxed in acetonitrile (3ml) for 3 hours, cooled, poured on 1N HCl and extracted with ethyl acetate twice. The aqueous phase is made basic by adding a saturated solution of NaHCO₃ and extracted with ethyl acetate three times. The combined organic phases are dried over Na₂SO₄, evaporated to dryness yielding the title compound as slightly colored crystals (208mg; 25%).
1H-NMR (400MHz; DMSO): 2.50 (bt, 4H); 2.73 (bt, 2H); 3.59 (bt, 4H); 4.25 (t, 2H); 7.22 (s, 1H); 7.27 (s, 1H); 10.17 (s, 1H).
MS (m/z) Cm: 286 (MH+, 100).

### b) 2-Chloro-5-methoxy-4-(2-N-morpholinylethyloxy)benzaldehyde (3)

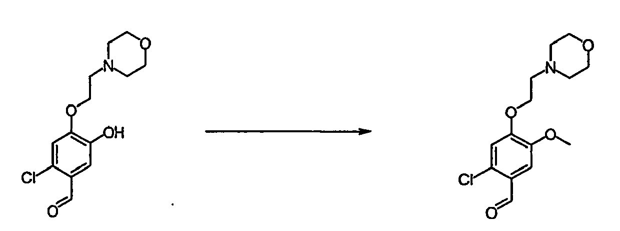

2-Chloro-5-hydroxy-4-(2-N-morpholinylethyloxy)benzaldehyde (2.54g: 8.9mmol) in acetone (60ml) is refluxed for 1 hour with MeI (0.56ml; 8.9mml) and K₂CO₃ (1.2g; 8.9mmol). The reaction mixture is diluted with water (100ml) and 2N NaOH (20ml) and extracted with ethyl acetate three times. The combined organic phases are dried over Na₂SO₄, evaporated to dryness and purified via chromatography (SiO₂; acetone/hexanes 4/6 > 1/1) to yield the title compound as yellow crystals (850mg; 32%)
1H-NMR (400MHz; DMSO): 2.50 (m, 4H); 2.73 (t, 2H); 3.58 (t, 4H); 3.83 (s, 3H); 4.23 (t, 2H); 7.26 (s, 1H); 7.35 (s, 1H); 10.20 (s, 1H).
MS (m/z) Cm: 286 (MH+, 100).

### Preparation of 2-methoxy-5-N-morpholinylbenzaldehyde (4)

### a) 2-(1,3-dioxolan-2-yl)-4-N-morpholinylanisole

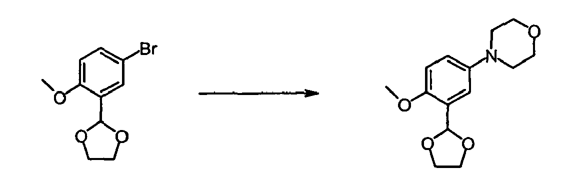

2-(1,3-Dioxolan-2-yl)-4-bromoanisole (Hall C. et al: J.Organomet.Chem. 1998, 561(1-2), 209-219) (2.6g; 10mmol), morpholine (2.1g; 24mmol), NaOtBu (2.7g; 28mmol), Pd2(dba)3 (46mg; 0.05mmol) and tri-o-tolylphosphine (61mg; 0.2mmol) are refluxed in dioxane (40ml) for 1 hour poured on water and extracted with ethyl acetate three times. The combined organic phases are washed with water, dried over Na₂SO₄, evaporated to dryness and purified via chromatography (SiO₂; cyclohexane/acetone 85/15) to yield the title compound as yellow crystals (500mg; 19%). 1H-NMR (400MHz; DMSO): 3.00 (bt, 4H); 3.77 (s, 3H); 3.78 (bt, 4H); 3.92 (m, 2H); 4.06 (m, 2H); 5.95 (s, 1H); 6.96 (s, 2H); 7.03 (s, 1H).
MS (m/z) EI: 265 (M+, 100); 250 (5); 207 (25); 135 (50).

### b) 2-Methoxy-5-N-morpholinylbenzaldehyde (4)

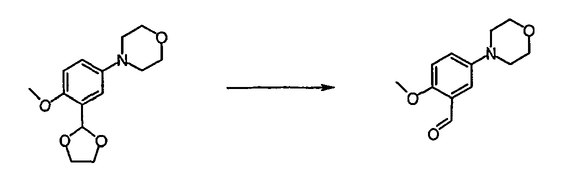

2-(1,3-Dioxolan-2-yl)-4-N-morpholinylanisole (265mg; 1mmol) is treated with acetone/H₂SO₄ (10ml / 215mg) for 10min at room temperature. The reaction mixture is poured on 2N Na₂CO₃ and extracted with ethyl acetate twice. The combined organic phases are washed with water, dried over Na₂SO₄, evaporated to dryness and purified via chromatography (SiO₂; cyclohexane/acetone 85/15) to yield the title compound as yellow oil (200mg; 90%).
1H-NMR (400MHz; DMSO): 3.08 (bt, 4H); 3.76 (bt, 4H); 3.88 (s, 3H); 7.19 (d, 1H); 7.20 (s, 1H); 7.47 (dd, 1H); 10.35 (s, 1H).
MS (m/z) EI: 221 (M+; 100); 206 (25); 163 (80); 148 (20); 134 (20); 117 (15).

### Preparation of 2-methoxy-5-(4-pyridinyl)benzaldehyde (5)

### a) 2-(1,3-Dioxolan-2-yl)-4-(4-pyridinyl)anisole

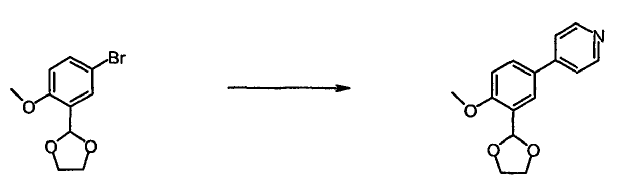

2-(1,3-Dioxolan-2-yl)-4-bromoanisole (Hall C. et al: J.Organomet.Chem. 1998, 561(1-2), 209-219)(500mg; 0.19mmol) dissolved in xylene (10ml) is combined with 4-trimethylstannylpyridine (560mg; 0.23mmol) and PdCl₂(PPh₃)₂ (140mg; 0.019mmol) and refluxed for 15min. The reaction mixture is diluted with toluene, decanted and purified via chromatography (SiO₂; acetone/hexanes 2/8 > 4/6) to yield the title compound (346mg; 70%) as a viscuous oil.
1H-NMR (400MHz; DMSO): 3.87 (s, 3H); 3.95 (m, 2H); 4.10 (m, 2H); 6.03 (s, 1H); 7.20 (d, 1H); 7.67 (d, 2H); 7.82 (d, 1H); 7.85 (d, 1H); 8.58 (d, 2H).
MS (m/z) EI: 257 (M+, 100).

### b) 2-Methoxy-5-(4-pyridinyl)benzaldehyde (5)

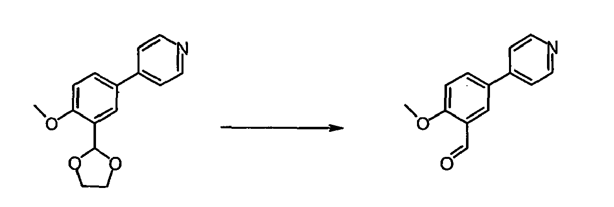

2-(1,3-Dioxolan-2-yl)-4-(4-pyridinyl)anisole (100mg; 0.39mmol) is refluxed with acetone/H₂SO₄conc (6ml/90mg; 0.8mmol) for 10min. The reaction mixture is poured on 2N Na₂CO₃ and extracted with ethyl acetate three times. The combined organic phases are washed with water, dried over Na₂SO₄, evaporated to dryness and yielded the title compound as a yellow oil (79mg; 95%).
1H-NMR (400MHz; DMSO): 4.02 (s, 3H); 7.43 (d, 1H); 7.75 (d, 2H); 8.10 (d, 1H); 8.17 (dd, 1H); 8.63 (d, 2H); 10.42 (s, 1H).
MS (m/z) EI: 213 (M+, 100); 196 (30); 167 (25); 115 (15).

### 5-(1,2-Dimethyl-1.H.-imidazol-4-yl)-2-methoxy-benzaldehyde (8)

### a) 4-(3-[1,3]Dioxolan-2-yl-4-methoxy-phenyl)-1,2-dimethyl-1.H.-imidazole

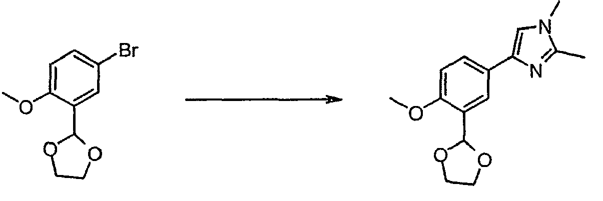

1,2-Dimethyl-1.H.-imidazole (4.8g, 50mmol), 2-(5-bromo-2-methoxy-phenyl)-[1,3]dioxolane (6.5g, 25mmol), Pd(OAc)₂ (140mg, 0.625mmol), PPh₃ (327mg, 1.25mmol) and Cs₂CO₃ (8.15g; 25mmol) are dissolved in DMF (50ml) and heated to 145C for 5h under argon. The reaction mixture is poured on saturated NaCl-solution and extracted with EtOAc 3 times. The organic phases are dried over Na₂SO₄, evaporated to dryness and purified via chromatography (SiO₂, Acetone/EtOH 9/1) to yield the title compound as a clear oil (4.0g; 58%).
1H-NMR (400MHz; DMSO-d6): 2.33 (s, 3H); 3.46 (s, 3H); 3.82 (s, 3H); 3.92 (m, 2H); 4.05 (m, 2H); 6.02 (s, 1H); 6.78 (s, 1H); 7.12 (d, 1H); 7.40 (m, 2H).
MS (m/z) ES+: 275 (MH+, 100).

### b) 5-(1,2-Dimethyl-1.H.-imidazol-4-yl)-2-methoxy-benzaldehyde

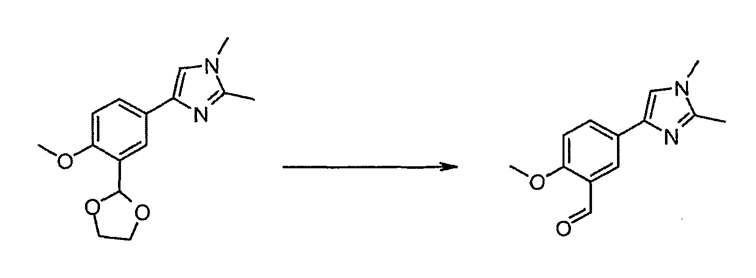

4-(3-[1,3]Dioxolan-2-yl-4-methoxy-phenyl)-1,2-dimethyl-1.H.-imidazole (4.0g, 14.5mmol) is dissolved in acetone/H₂SO₄conc (264ml/3.2g) and stirred for 3h at room temperature. Acetone is partially evaporated and the residue dissolved in EtOAc, washed with 2N Na₂CO₃ and water, dried over Na₂SO₄ and evaporated to dryness. The crude product is recrystallised from TBME/hexanes to yield the title compound as colorless crystals (2.8g, 83%).
1H-NMR (400MHz; DMSO-d6): 2.34 (s, 3H); 3.49 (s, 3H); 3.96 (s, 3H); 6.85 (s, 1H); 7.34 (d, 1H); 7.66 (d, 1H); 7.73 (dd, 1H); 10.38 (s, 1H).
MS (m/z) EI: 230 (M+, 100), 215 (10(; 187 (40).

Aldehydes 1-6 are converted into the compounds of Examples 6-11 according to scheme 1 using the general procedure described for example 1.

### Example 6: [3-(2-Chloro-4,5-dimethoxy-phenyl)-1.H.-pyrazolo[3,4-.b.]pyridin-6-yl]-(2,4-difluoro-phenyl)-amine

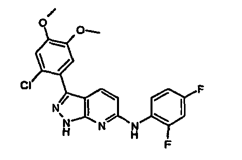

1H-NMR (400MHz; DMSO): 3.80 (s, 3H); 3.87 (s, 3H); 6.83 (d, 1H); 7.11 (m, 2H); 7.19 (s, 1H); 7.35 (bt, 1H); 7.84 (d, 1H); 8.17 (m, 1H); 9.03 (s, 1H); 13.20 (s, 1H).
MS (m/z) Cm: 415 (MH-, 100); 367 (50); 322 (40); 255 (15); 209 (80).

### Example 7: (2,4-Difluoro-phenyl)-[3-(2-methoxy-5-morpholin-4-yl-phenyl)-1.H.-pyrazolo[3,4-.b.]pyridin-6-yl]-amine

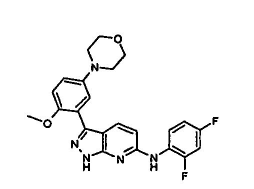

1H-NMR (400MHz; DMSO): 3.03 (m, 4H); 3.78 (m, 7H); 6.80 (d, 1H); 7.03 (dd, 1H); 7.10 (m, 2H); 7.18 (d, 1H); 7.33 (m, 1H); 7.87 (d, 1H); 8.18 (m, 1H); 8.97 (s, 1H); 13.2 (s, 1H).
MS (m/z) ES: 438 (M+, 100); 330 (20).

### Example 8: (2,4-Difluoro-phenyl)-[3-(2-methoxy-5-pyridin-4-yl-phenyl)-1.H.-pyrazolo[3,4-.b.]pyridin-6-yl]-amine

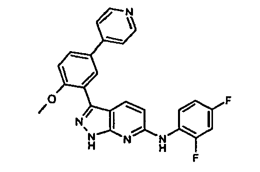

1H-NMR (400MHz; DMSO): 3.93 (s, 3H); 6.82 (d, 1H); 7.12 (m, 1H); 7.33 (m, 2H); 7.75 (d, 2H); 7.91 (m, 2H); 8.00 (d 1H); 8.20 (m, 1H); 8.62 (d, 2H); 9.01 (s, 1H); 13.2 (s, 1H).
MS (m/z) ES: 430 (H+, 100); 317 (10).

### Example 9: {3-[2-Chloro-5-methoxy-4-(2-morpholin-4-yl-ethoxy)-phenyl]-1.H.-pyrazolo[3,4-.b.]pyridin-6-yl}-(2,4-difluoro-phenyl)-amine

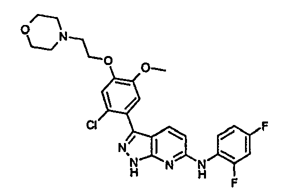

1H-NMR (400MHz; DMSO): 2.53 (bs, 4H); 2.75 (t, 2H); 3.62 (t, 4H); 3.82 (s, 3H); 4.20 (t, 2H); 6.84 (d, 1H); 7.10 (s, 1H); 7.13 (bd, 1H); 7.24 (s, 1H); 7.35 (dt, 1H); 7.84 (d, 1H); 8.17 (m, 1H); 9.05 (s, 1H); 13.10 (s, 1H).
MS (m/z) ES+: 516 (MH+, 100).

### Example 10: {3-[2-Chloro-4-methoxy-5-(2-morpholin-4-yl-ethoxy)-phenyl]-1.H-pyrazolo[3,4-.b.]pyridin-6-yl}-(2,4-difluoro-phenyl)-amine

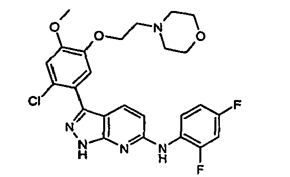

1H-NMR (400MHz; DMSO): 2.47 (t, 4H); 2.70 (t, 2H); 3.58 (t, 4H); 3.88 (s, 3H); 4.13 (t, 2H); 6.83 (d, 1H); 7.12 (bd, 1H); 7.16 (s, 1H); 7.19 (s, 1H); 7.33 (m, 1H); 7.84 (d, 1H); 8.18 (m, 1H); 9.05 (s, 1H); 13.20 (2, 1H).
MS (m/z) ES+: 516 (MH+, 100).

### Example 11: (2,4-Difluoro-phenyl)-{3-[5-(1,2-dimethyl-1.H.-imidazol-4-yl)-2-methoxy-phenyl]-1.H.-pyrazolo[3.4-.b.]pyridin-6-yl}-amine

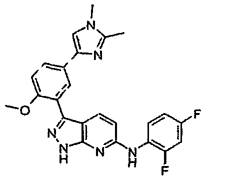

1H-NMR (400MHz; DMSO): 2.33 (s, 3H); 3.52 (s, 3H); 3.87 (s, 3H); 6.78 (d, 1H); 6.82 (s, 1H); 7.09 (bt, 1H); 7.26 (d, 1H); 7.34 (dt, 1H); 7.43 (dd, 1H); 7.57 (d, 1H); 7.87 (d, 1H); 8.18 (m, 1H); 8.98 (s, 1H, NH); 13.12 (s, 1H, NH).
MS (m/z) ES+: 447 (MH+, 100).

The common precursor for the compounds in Example 12-22 (scheme 3) is bromide **6**, which is prepared according to scheme 1 from commercially available 5-bromo-2-methoxybenzaldehyde and 2,6-dichloropyridine in 3 steps following the general procedure described for Example 1c.

Compound **6** is converted according to scheme 3 into aldehyde 7, which serves as precursor for the amines in Examples 13-18. Compound **6** is further transformed into the piperazine analogue in Example 22 and into the primary amines in Examples 20 and 21.

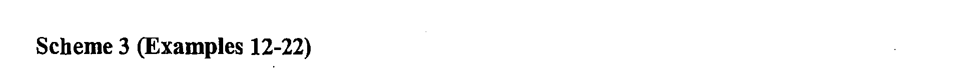

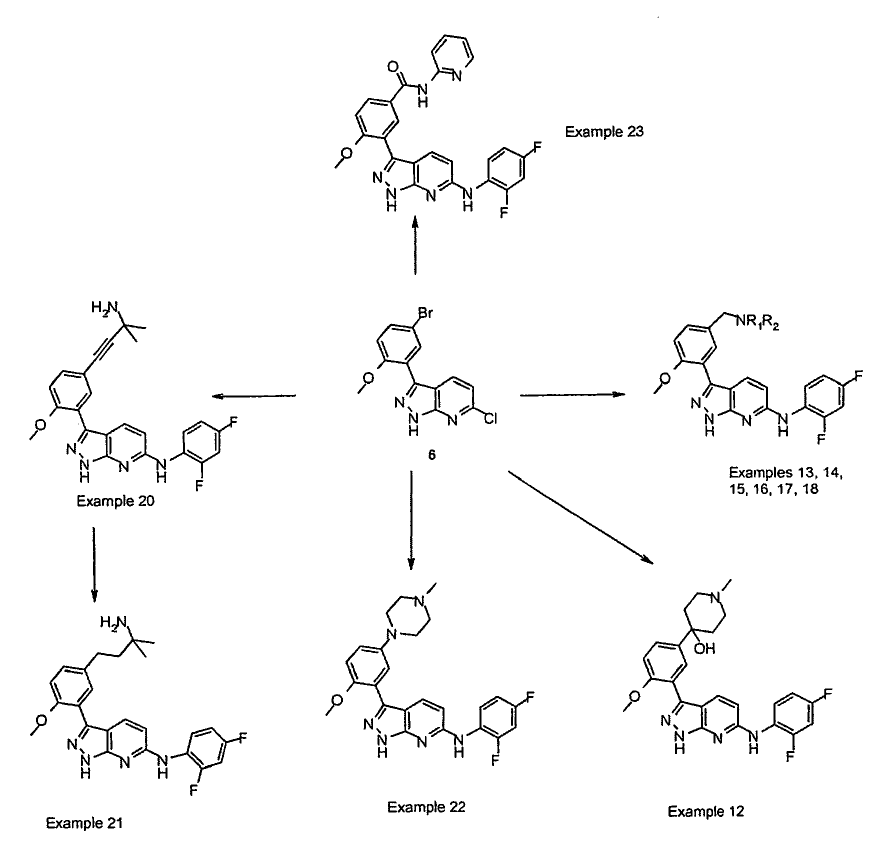

### Compound 6: 3-(5-Bromo-2-methoxy-phenyl)-6-chloro-1.H.-pyrazolo[3,4-.b.]pyridine

Prepared from commercially available 5-bromo-2-methoxybenzaldehyde in three steps according to scheme 1 following the general procedure described for 6-chloro-3-(2-methoxy-phenyl)-1.H.-pyrazolo[3,4-.b.]pyridine (1c).
1H-NMR (400MHz; DMSO): 3.87 (s, 3H); 7.21 (d, 1H); 7.29 (d, 1H); 7.63 (dd, 1H); 7.78 (d, 1H); 8.25 (d, 1H).
MS (m/z) Cm: 338 (MH-, 100); 255 (20); 173 (25); 155 (50).

### Example 12: 4-{3-[6-(2,4-Difluoro-phenylamino)-1.H.-pyrazolo[3,4-.b.]pyridin-3-yl]-4-methoxy-phenyl}-1-methyl-piperidin-4-ol

### a) 4-[3-(6-Chloro-1.H.-pyrazolo[3,4-.b.]pyridin-3-yl)-4-methoxy-phenyl]-1-methyl-piperidin-4-ol

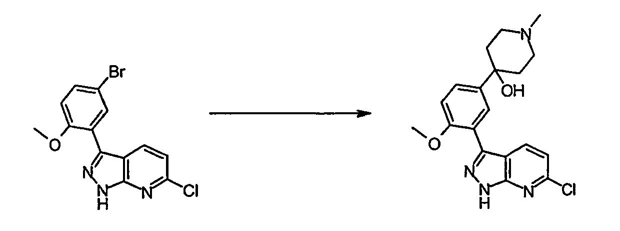

nBuLi (1.46ml; 2.33mmol of a 1.6M solution in hexane) is slowly added at -78°C to a solution of 3-(5-bromo-2-methoxy-phenyl)-6-chloro-1.H.-pyrazolo[3,4-.b.]pyridine (compound **6**)(400mg; 1.2mmol) in THF (10ml). The yellow solution is stirred at -78°C for 10min, then N-methyl-4-piperidone (0.272ml; 2.4mmol) in THF (0.5ml) is added rapidly. After stirring for another 10min at -78°C, the reaction mixture is poured on an aqueous NaCl solution and extracted with ethyl acetate three times. The combined organic phases are washed with water, dried over Na₂SO₄, evaporated to dryness and yielded the title compound as a yellow foam, which crystallised upon taking up in ether (280mg; 62%).
1H-NMR (400MHz; DMSO): 1.63 (bd, 2H); 1.97 (dt, 2H); 2.22 (s, 3H); 2.37 (bt, 2H); 2.53 (bt, 2H); 3.83 (s, 3H); 4.77 (s, 1H, OH); 7.15 (d, 1H); 7.20 (bd, 1H); 7.51 (dd, 1H); 7.78 (d, 1H); 8.19 (d, 1H).
MS (m/z) ES+: 373 (MH+; 100).

### b) 4-{3-[6-(2,4-Difluorophenylamino)-1.H.-pyrazolo[3,4-.b.]pyridin-3-yl]-4-methoxy-phenyl}-1-methyl-piperidin-4-ol

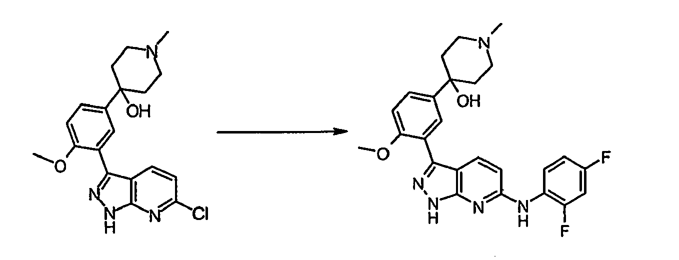

4-[3-(6-Chloro-1.H.-pyrazolo[3,4-.b.]pyridin-3-yl)-4-methoxy-phenyl]-1-methyl-piperidin-4-ol (280mg; 0.75mmol) and 2,4-difluoroaniline (3ml; 29mmol) are dissolved in hot dioxane, combined with NaOtBu (280mg; 2.9mmol), Pd(OAc)₂ (25mg; 0.11mol) and R-(+)-BINAP (25mg; 0.04mmol) and refluxed for 30min. The reaction mixture is poured on water/NaCl and extracted with ethyl acetate three times. The combined organic phases are washed with water, dried over Na₂SO₄, evaporated to dryness and purified via chromatography (SiO₂; TBME/MeOH/NH₃conc 80/20/2) to yield the title compound as brownish crystals (180mg; 52%) 1H-NMR (400MHz; DMSO): 1.64 (bd, 2H); 1.96 (dt, 2H); 2.22 (s, 3H); 2.37 (bt, 2H); 2.53 (bt, 2H); 3.83 (s, 3H); 4.73 (s, 1H); 6.80 (d, 1H); 7.11 (m, 2H); 7.33 (dt, 1H); 7.48 (dd, 1H); 7.72 (d, 1H); 7.87 (d, 1H); 8.20 (m, 1H); 8.97 (s, 1H); 13.10 (s, 1H).
MS (m/z) ES+: 466 (MH+, 100).

### Example 13: (2,4-Difluoro-phenyl)-[3-(2-methoxy-5-methylaminomethyl-phenyl)-1.H.-pyrazolo[3,4-.b.]pyridin-6-yl]-amine

### a) 3-(6-Chloro-1.H.-pyrazolo[3,4-.b.]pyridin-3-yl)-4-methoxy-benzaldehyde

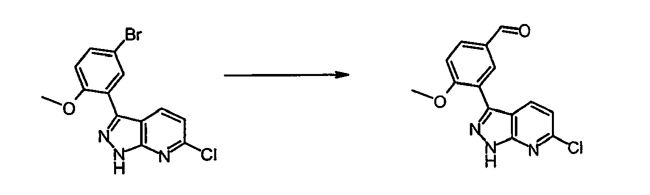

3-(5-Bromo-2-methoxy-phenyl)-6-chloro-1.H.-pyrazolo[3,4-.b.]pyridine_(compound 6) (1g; 2.9mmol) in THF (50ml) is cooled to -78°C and treated with nBuLi (3.7ml; 5.9mml of a 1.6M solution in hexane). After 10min at -78°C DMF (0.455ml; 6.6mmol) is introduced and stirring conrinued for 10min. The reaction mixture is warmed to -30C, poured on water /NaCl and extracted with TBME three times. The combined organic phases are washed with water, dried over Na₂SO₄, evaporated to dryness and purified via chromatography (SiO2; acetone/hexanes 1/9 > 2/8) to yield the desired aldehyde (270mg; 31 %) as colorless crystals and debrominated starting material (330mg)
1H-NMR (400MHz; DMSO): 4.00 (s, 3H); 7.31 (d, 1H); 7.46 (d, 1H); 8.06 (dd, 1H); 8.21 (s, 1H); 8.27 (d, 1H); 10.00 (s, 1H); 14.10 (s, 1H, NH).
MS (m/z) EI: 287 (M+, 100); 271 (15); 258 (25).

### b) [3-(6-Chloro-1.H.-pyrazolo[3.4-.b.]pyridin-3-yl)-4-methoxy-benzyl]-methyl-amine

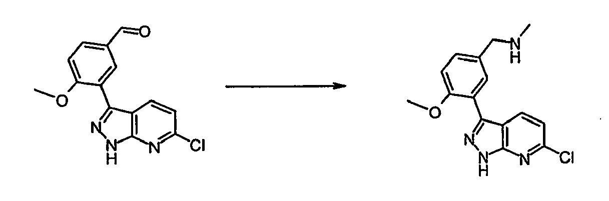

3-(6-Chloro-1.H.-pyrazolo[3,4-.b.]pyridin-3-yl)-4-methoxy-benzaldehyde (270mg; 0.94mmol) is dissolved in EtOH (75ml), warmed to 40°C and combined with a 10% solution of MeNH₂ in EtOH (4.5ml). After stirring at 40°C for 2min, NaBH₄ (54mg; 1.4mmol) in EtOH (2ml) is added and stirring continued for 5min. The reaction mixture was poured on water/NaCl and extracted with TBME three times. The combined organic phases are washed with water, dried over Na₂SO₄, evaporated to dryness and purified via chromatography (SiO₂; TBME/MeOH/NH₃conc 80(18/2) to yield the title compound as a colorless foam (140mg; 49%). 1H-NMR (400MHz; DMSO): 2.31 (s, 3H); 3.68 (s, 2H); 3.85 (s, 3H); 7.18 (bt, 1H); 7.25 (s, 1H); 7.43 (bs, 1H); 7.60 (s, 1H); 8.22 (s, 1H).
MS (m/z) EI: 302 (M+, 40); 272 (50); 139 (40).

### c) (2,4-Difluoro-phenyl)-[3-(2-methoxy-5-methylaminomethyl-phenyl)-1.H.-pyrazolo[3,4-.b.]pyridin-6-yl]-amine

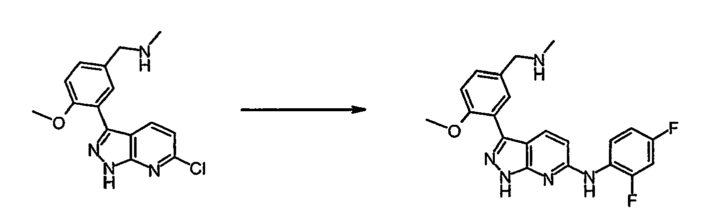

The Buchwald reaction is carried out by analogy to Example 12b) and yields the desired compound as a colorless foam in 10% yield. 1H-NMR (400MHz; DMSO): 2.28 (s, 3H); 3.63 (s, 2H); 3.82 (s, 3H); 6.80 (d, 1H); 7.11 (m, 2H); 7.32 (m, 2H); 7.54 (d, 1H); 7.88 (d, 1H); 8.20 (m, 1H); 8.98 (s, 1H); 13.05 (s, 1H).
MS (m/z) ES+: 396 (MH+, 20); 365 (100).
Compounds of Examples 14-18 are prepared from 3-(6-Chloro-1.H.-pyrazolo[3,4-.b.]pyridin-3-yl)-4-methoxy-benzaldehyde (Example 13a) by a reductive amination followed by a Buchwald reaction by analogy to Example 13:

### Example 14: (2,4-Difluoro-phenyl)-{3-[2-methoxy-5-(4-methyl-piperazin-1-ylmethyl)-phenyl]-1.H.-pyrazolo[3,4-.b.]pyridin-6-yl}-amine

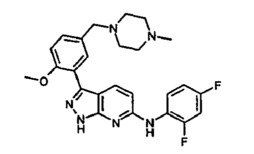

1H-NMR (400MHz; DMSO): 2.17 (s, 3H); 2.24-2.48 (m, 8H); 3.43 (s, 2H); 3.82 (s, 3H); 6.80 (d, 1H); 7.11 (m, 2H); 7.29-7.38 (m, 2H); 7.53 (s, 1H); 7.88 (d, 1H); 8.18 (m, 1H); 8.98 (s, 1H); 13.2 (s, 1H).
MS (m/z) ES+: 465 (MH+, 100); 365 (20).

### Example 15: (2,4-Difluoro-phenyl)-[3-(2-methoxy-5-morpholin-4-ylmethyl-phenyl)-1.H.-pyrazolo[3,4-.b.]pyridin-6-yl]-amine

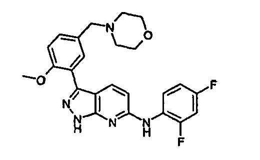

1H-NMR (400MHz; DMSO): 2.38 (bs, 4H); 3.47 (s, 2H); 3.59 (bs, 4H); 3.84 (s, 3H); 6.80 (d, 1H); 7.12 (m, 2H); 7.33 (m, 2H); 7.56 (d, 1H); 7.88 (d, 1H); 8.19 (m, 1H); 8.97 (s, 1H); 13.2 (s,
1H).
MS (m/z) ES+: 452 (MH+, 100); 365 (50); 330 (10); 289 (10).

### Example 16: (2,4-Difluoro-phenyl)-[3-(2-methoxy-5-piperazin-1-ylmethyl-phenyl)-1.H.-pyrazolo[3,4-.b.]pyridin-6-yl]-amine

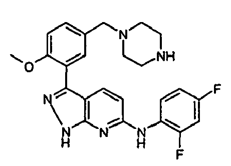

1H-NMR (400MHz; DMSO): 2.31 (m, 4H); 2.67 (m, 4H); 3.41 (s, 2H); 3.83 (s, 3H); 6.80 (d, 1H); 7.11 (m, 2H); 7.28-7.37 (m, 2H); 7.52 (d, 1H); 7.88 (d, 1H); 8.18 (m, 1H); 8.97 (s, 1H); 13.10 (s, 1H).
MS (m/z) ES+: 451 (MH+, 100); 365 (20).

### Example 17: (2,4-Difluoro-phenyl)-[3-(2-methoxy-5-propylaminomethyl-phenyl)-1.H.-pyrazolo[3,4-.b.]pyridin-6-yl]-amine

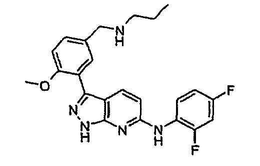

1H-NMR (400MHz; DMSO): 0.88 (t, 3H); 1.44 (q, 2H); 2.47 (t, 2H); 3.69 (s, 2H); 3.83 (s, 3H); 6.82 (d, 1H); 7.12 (m, 2H); 7.35 (m, 2H); 7.56 (d, 1H); 7.88 (d, 1H); 8.21 (m, 1H); 8.98 (s, 1H, NH); 13.09 (s, 1H, NH).
MS (m/z) ES+: 422 (MH+, 100).

### Example 18: (2,4-Difluoro-phenyl)-(3-{2-methoxy-5-[(tetrahydro-pyran-4-ylamino)-methyl]-phenyl}-1.H.-pyrazolo[3,4-.b.]pyridin-6-yl)-amine

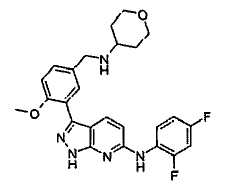

1H-NMR (400MHz; DMSO): 1.22-1.47 (m, 2H); 1.82 (bd, 2H); 2.63 (m, 1H); 3.30 (m, 2H); 3.75 (bs, 2H); 3.87 (m, 2H); 3.83 (s, 3H); 6.81 (d, 1H); 7.12 (m, 2H); 7.31-7.40 (m, 2H); 7.58 (d, 1H); 7.88 (d, 1H); 8.13-8.23 (m, 1H); 8.98 (s, 1H, NH); 13.09 (s, 1H, NH).
MS (m/z) ES+: 466 (MH+, 100).

### Example 19: (2,4-Difluoro-phenyl)-{3-[5-(1,2-dimethyl-1.H.-imidazol-4-yl)-2-methoxy-phenyl]-1.H.-pyrazolo[3,4-.b.]pyridin-6-yl}-amine

(2,4-Difluoro-phenyl)-{3-[5-(1,2-dimethyl-1.H.-imidazol-4-yl)-2-methoxy-phenyl]-1.H.-pyrazolo[3,4-.b.]pyridin-6-yl} is prepared in four steps from 5-(1,2-Dimethyl-1.H.-imidazol-4-yl)-2-methoxy-benzaldehyde (compound **8**) and 2,6-dichloropyridine as described in examples 1a)-1d) 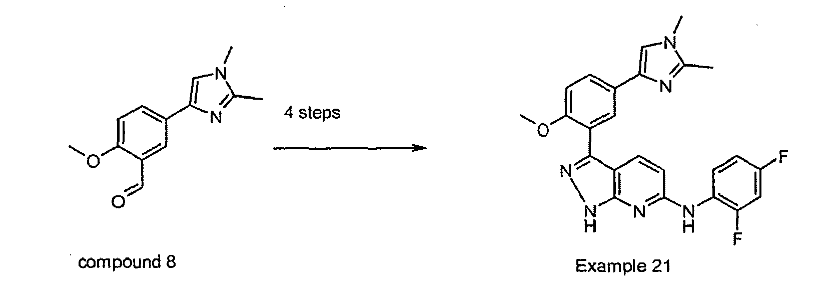 1H-NMR (400MHz; DMSO): 2.33 (s, 3H); 3.52 (s, 3H); 3.87 (s, 3H); 6.78 (d, 1H); 6.82 (s, 1H); 7.08 (bt, 1H); 7.26 (d, 1H); 7.33 (dt, 1H); 7.43 (dd, 1H); 7.57 (5, 1H); 7.87 (d, 1H); 8.13 (m, 1H); 8.98 (s, 1H, NH); 13.12 (s, 1H, NH).
MS (m/z) ES+: 447 (MH+, 100).

Example 20 (scheme 3) is prepared in two steps from 3-(5-bromo-2-methoxy-phenyl)-6-chloro-1.H.-pyrazolo[3,4-.b.]pyridine (compound 6) by a Sonogashira coupling with 3-amino-3-methyl-1-butyne followed by a Buchwald reaction. Hydrogenation of the triple bond delivered the compound of Example 21:

### Example 20: {3-[5-(3-Amino-3-methyl-but-1-ynyl)-2-methoxy-phenyl]-1.H.-pyrazolo[3,4-.b.]pyridin-6-yl}-(2,4-difluoro-phenyl)-amine

### a) 3-[3-(6-Chloro-1.H.-pyrazolo[3,4-.b.]pyridin-3-yl)-4-methoxy-phenyl]-1,1-dimethyl-prop-2-ynylamine

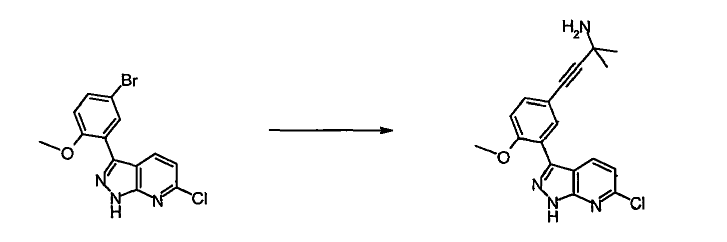

3-(5-Bromo-2-methoxy-phenyl)-6-chloro-1.H.-pyrazolo[3,4-.b.]pyridine (compound **6**)(1g; 2.94mmol), 3-amino-3-methyl-1-butyne (0.35ml; 3.3mmol), PdCl₂(PPh3)2 (210mg; 0.29mmol) and CuI (172mg; 0.29mmol) are refluxed in triethylamine (140ml) and dioxane (30ml) for 3 hours. The reaction mixture is filtered, evaporated and purified via chromatography (SiO₂; TBME/MeOH/NH₃conc 90/9/1) to yield the title compound as a yellow foam (218mg; 20%). 1H-NMR (400MHz; DMSO): 1.40 (s, 6H); 3.88 (s, 3H); 7.20 (d, 1H); 7.28 (d, 1H); 7.47 (dd, 1H); 7.63 (d, 1H); 8.23 (d, 1H);
MS (m/z) EI: 340 (M+, 10); 325 (100), 277 (20).

### b) {3-[5-(3-Amino-3-methyl-but-1-ynyl)-2-methoxy-phenyl]-1.H.-pyrazolo[3,4-.b.]pyridin-6-yl}-(2,4-difluoro-phenyl)-amine

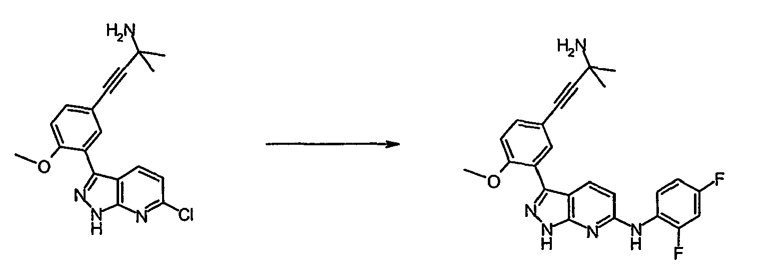

3-[3-(6-Chloro-1.H.-pyrazolo[3,4-.b.]pyridin-3-yl)-4-methoxy-phenyl]-1,1-dimethyl-prop-2-ynylamine (350mg; 1.02mmol) in dioxane (7ml) is combined with 2,4-difluoroaniline (3ml; 19.3mmol), NaOtBu (350mg; 3.6mmol), Pd(OAc)₂ (70mg; 0.308mol) and R-(+)-BINAP (70mg; 0.112mmol) and refluxed for 1 hour. The reaction mixture is poured on 2N HCl and extracted with ethyl acetate three times. The combined organic phases are made basic with 2N NaOH and extracted three times with ethyl acetate. The combined organic phases are dried over Na₂SO₄ and evaporated to dryness. The residue is further purified by dissolving in hot toluene and filtering off the dark precipitate formed after cooling. Toluene is evaporated and the residue recrystallised from ethyl acetate resulting in the title compound as off-white crystals (20mg; 6%). 1H-NMR (400MHz; DMSO): 1.39 (s, 6H); 2.06 (bs, 2H); 3.80 (s, 3H); 6.81 (d, 1H); 7.11 (bd, 1H); 7.15 (d, 1H); 7.34 (dt, 1H); 7.40 (d, 1H); 7.59 (d, 1H); 7.88 (d, 1H); 8.18 (m, 1H); 9.00 (s, 1H); 13.17 (s, 1H).
MS (m/z) ES+: 434 (MH+, 70); 417 (100).

### Example 21: {3-[5-(3-Amino-3-methyl-butyl)-2-methoxy-phenyl]-1.H.-pyrazolo[3,4-.b.]pyridin-6-yl}-(2,4-difluoro-phenyl)-amine

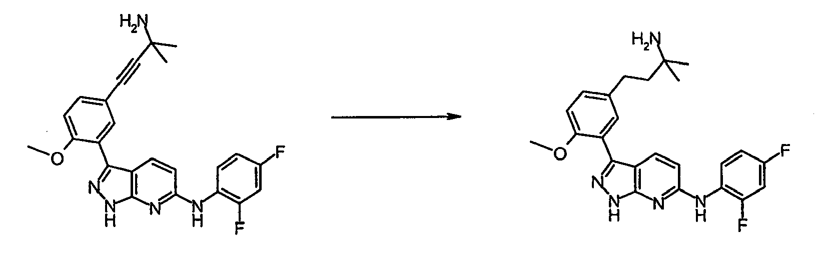

{3-[5-(3-Amino-3-methyl-but-1-ynyl)-2-methoxy-phenyl]-1.H.-pyrazolo[3,4-.b.]pyridin-6-yl}-(2,4-difluoro-phenyl)-amine (50mg; 0.11mmol) is dissolved in EtOH (100ml) and hydrogenated over 10% Pd/C (100mg) for 1 hour. The reaction mixture is filtered, evaporated and yields the title compound as colorless crystals (30mg; 60%) after recrystallisation from ether/hexanes. 1H-NMR (400MHz; DMSO): 1.08 (s, 6H); 1.53-1.60 (m, 4H); 2.58-2.63 (m, 2H); 3.81 (s, 3H); 6.80 (d, 1H); 7.03-7.14 (m, 2H); 7.22 (d, 1H); 7.32 (bt, 1H); 7.43 (s, 1H); 7.87 (d, 1H); 8.19 (m, 1H); 8.90 (s, 1H); 13.10 (s, 1H).
MS (m/z) ES+: 438 (MH+, 100); 421 (20); 192 (10); 180 (50).

### Example 22: (2,4-Difluoro-phenyl)-{3-[2-methoxy-5-(4-methyl-piperazin-1-yl)-phenyl]-1.H.-pyrazolo[3,4-.b.]pyidin-6-yl}-amine

### a) 6-Chloro-3-[2-methoxy-5(4-methyl-piperazin-1-yl)-phenyl]-1.H.-pyrazolo[3.4.b.]pyridine

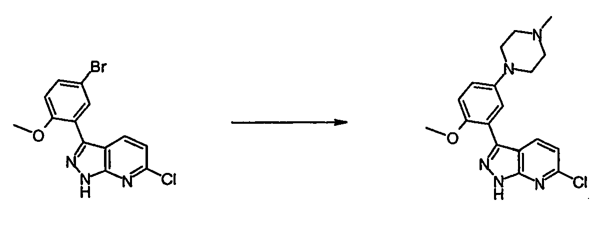

3-(5-Bromo-2-methoxy-phenyl)-6-chloro-1.H.-pyrazolo[3,4-.b.]pyridine (compound 6) (200mg; 0.588mmol) NaOtBu (224mg; 2.3mmol), Pd(OAc)2 (20mg; 0.08mol) and R-(+)-BINAP (20mg; 0.03mmol) and N-methylpiperazine (0.1ml; 1mmol) are refluxed in dioxane (4ml) for 3 hours. TBME (20ml) is added to the reaction mixture, filtered from the precipitate and purified via chromatography (SiO₂; TBME/MeOH/NH₃conc 90/10/1) to yield the title compound (40mg; 19%) after recrystallisation from ether/hexanes.
1H-NMR (400MHz; DMSO): 2.24 (s, 3H); 2.48 (bt, 4H); 3.10 (bt, 4H); 3.78 (s, 3H); 7.05-7.11 (m, 2H); 7.20 (d, 1H); 7.28 (d, 1H); 8.20 (d, 1H); 13.90 (bs, 1H).
MS (m/z) Cm: 358 (MH+, 100); 324 (10).

### b) (2,4-Difluoro-phenyl)-{3-[2-methoxy-5-(4-methyl-piperazin-1-yl)-phenyl]-1.H.-pyrazolo[3,4-.b.]pyridin-6-yl}-amine (Example 8)

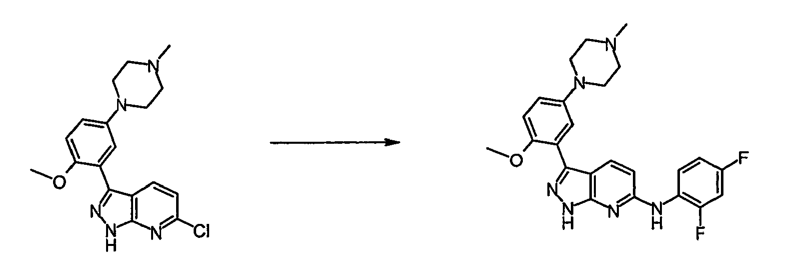

6-Chloro-3-[2-methoxy-5-(4-methyl-piperazin-1-yl)-phenyl]-1.H.-pyrazolo[3,4-.b.]pyridine (186mg; 0.52mmol) in dioxane (4ml) is combined with 2,4-difluoroaniline (3ml; 19.3mmol), NaOtBu (186mg; 1.9mmol), Pd(OAc)₂ (40mg; 0.175mol) and R-(+)-BINAP (40mg; 0.063mmol) and refluxed for 1 hour. The reaction mixture is taken up in 2N HCl and washed with ethyl acetate twice. The aqueous layer is made basic with 2N NaOH and extracted with ethyl acetate three times. The combined organic phases are dried over Na₂SO₄, filtered and evaporated to dryness to yield a dark residue, which is crystallised twice from hot toluene to render the title compound as slightly colored crystals (45mg; 20%). 1H-NMR (400MHz; DMSO): 2.23 (s, 3H); 2.45 (m, 4H); 3.07 (m, 4H); 3.77 (s, 3H); 6.79 (d, 1H); 7.01 (dd, 1H); 7.05-7.14 (m, 2H); 7.18 (d, 1H); 7.33 (m, 1H); 7.85 (d, 1H); 8.19 (m, 1H); 8.96 (s, 1H); 13.2 (s, 1H).
MS (m/z) Cm: 451 (MH+, 100).

### Example 23: 3-[6-(2,4-Difluoro-phenylamino)-1.H.-pyrazolo[3,4-.b.]pyridin-3-yl]-4-methoxy-.N.-pyridin-2-yl-benzamide

### a) 3-(5-Bromo-2-methoxy-pheyl)-6-chloro-1-(2-trimethylsilanyl-ethoxymethyl)-1.H.-pyrazolo[3,4-.b.]pyidine

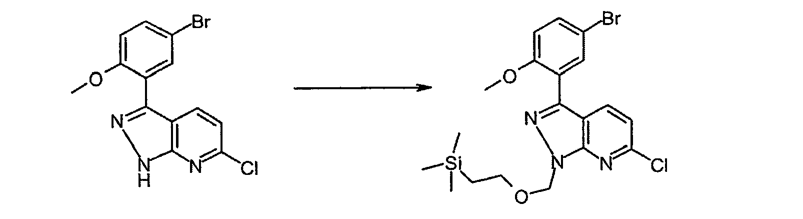

3-(5-Bromo-2-methoxy-phenyl)-6-chloro-1.H.-pyrazolo[3,4-.b.]pyridine (6.8g; 20mmol) is dissolved in THF (240ml) and treated at -78C with KN(TMS)2 (5.25g; 25mmol). After stirring for 15 minutes at -78C, (2-chloromethoxy-ethyl)-trimethyl-silane (4.47ml; 25mmol) is added and stirring continued for 15 minutes at -78C. The reaction mixture is warmed to 0C, poured on water/NaCl and extracted with TBME three times. The combined organic phases are dried over Na₂SO₄ and evaporated to dryness. Purification via chromatography (SiO₂ ; TBME/cyclohexane 10/90) yields the title compound as a colorless foam (6.0g; 64%), which is eluted before its isomer 3-(5-bromo-2-methoxy-phenyl)-6-chloro-2-(2-trimethylsilanyl-ethoxymethyl)-2.H.-pyrazolo[3,4-.b.]pyridine (3.2g; 34%).
1H-NMR (400MHz; DMSO): -0.08 (s, 9H); 0.88 (t, 2H); 3.68 (t, 2H); 3.88 (s, 3H); 5.81 (s, 2H); 7.23 (d, 1H); 7.40 (d, 1H); 7.68 (dd, 1H); 7.76 (d, 1H); 8.28 (d, 1H). MS (m/z) ES+: 470 (MH+; 100).

### b) 3-[6-Chloro-1-(2-trimethylsilanyl-ethoxymethyl)-1.H.-pyrazolo[3,4-.b.]pyridin-3-yl]-4-methoxy-benzoic acid

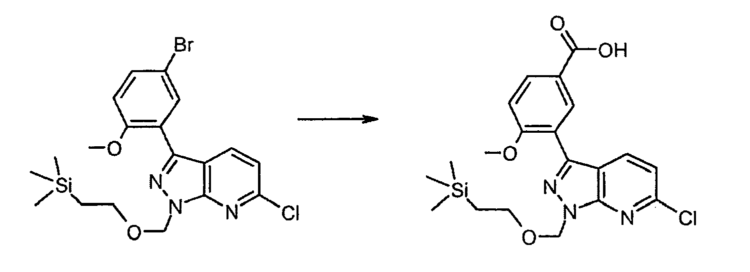

3-(5-Bromo-2-methoxy-phenyl)-6-chloro-1-(2-trimethylsilanyl-ethoxymethyl)-1.H.-pyrazolo[3,4-.b.]pyridine (3g; 6.38mmol) in THF (90ml) is cooled to -78C and treated with nBuLi (4.4ml; 7.02mmol of a 1.6M solution in hexane). Stirring is continued for 10 minutes, then CO₂-gas is introduced during 5 minutes. The reaction mixture is stirred for 10 minutes at -78C, then poured on 0.1 M HCl-solution and extracted with EtOAc three times. The organic phases are washed with brine, dried over Na₂SO₄ and evaporated to dryness. Purification via chromatography (SiO₂, acetone/hexanes/HOAc 20/80/1) yields the title compound (3.2g; 58%) as colorless crystals.
1H-NMR (400MHz; DMSO): -0.08 (s, 9H); 0.88 (t, 2H); 3.68 (t, 2H); 3.95 (s, 3H); 5.82 (s, 2H); 7.33 (d, 1H); 7.38 (d, 1H); 8.09 (dd, 1H); 8.22 (d, 1H); 8.30 (d, 1H).
MS (m/z) ES-: 432 (MH-; 100).

### c) 3-[6-(2,4-Difluoro-phenylamino)-1-(2-trimethylsilanyl-ethoxymethyl)-1.H.-pyrazolo[3,4-.b.]pyridin-3-yl]-4-methoxy-benzoic acid

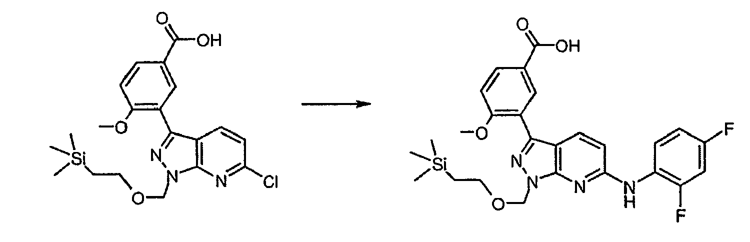

3-[6-Chloro-1-(2-trimethylsilanyl-ethoxymethyl)-1.H.-pyrazolo[3,4-.b.]pyridin-3-yl]-4-methoxybenzoic acid (3.2g; 7.39mmol), R-(+)-BINAP (229mg; 0.369mmol), Pd(OAc)₂ (248mg; 1.1mmol), PPh₃ (503mg; 1.9mmol), 2,4-difluoroaniline (18.7ml; 184mmol) and NaOtBu (1.77g; 18.47mmol) are dissolved in dioxane (50ml) and heated to 130C for 15 minutes. The reaction mixture is poured on brine and extracted with EtOAc twice. The combined organic phases are washed with 0.1N HCl and water, dried over Na₂SO₄ and evaporated to dryness. The crude product was purified via chromatography (SiO₂, Acetone/cyclohexane/HOAc 20/80/1) to yield the title compound as yellowish crystals (3.4g; 87%).
1H-NMR (400MHz; DMSO): -0.08 (s, 9H); 0.85 (t, 2H); 3.62 (t, 2H); 3.93 (s, 3H); 5.68 (s, 2H); 6.90 (d, 1H); 7.11 (dt, 1H); 7.28-7.38 (m, 2H); 7.92 (d, 1H); 8.03 (dd, 1H); 8.23 (d, 1H); 8.35-8.45 (m, 1H); 9.20 (s, 1H).
MS (m/z) ES-: 525 (MH-; 100).

### d) 3-[6-(2,4-Difluoro-phenylamino)-1-(2-trimethylsilanyl-ethoxymethyl)-1.H.-pyrazolo[3,4-.b.]pyridin-3-yl]-4-methoxy-.N.-pyridin-2-yl-benzamide

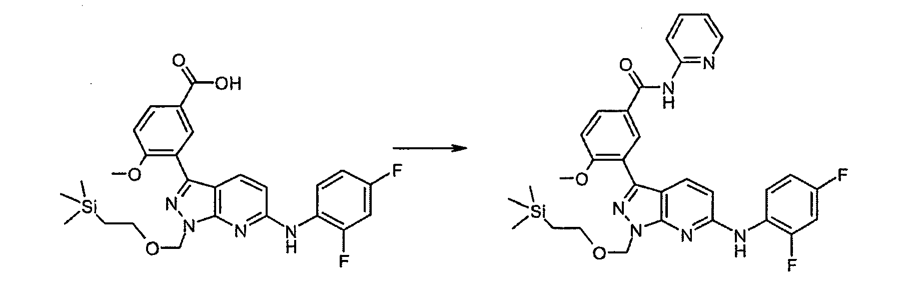

3-[6-(2,4-Difluoro-phenylamino)-1-(2-trimethylsilanyl-ethoxymethyl)-1.H.-pyrazolo[3,4-.b.]pyridin-3-yl]-4-methoxy-benzoic acid (1.1g; 2.08mmol) and 1,1'-carbonyldiimidazole (674mg; 4.16mmol) are dissolved in THF (7ml) and refluxed for 5 minutes until gas evolution ceases. The reaction mixture is cooled to 35C and 2-aminopyridine (1.17g; 12.48mmol) added. After heating at 80C for 5 minutes the reaction mixture is evaporated and kept at 130C for 30minutes. Purification via chromatography (SiO₂, acetone/hexanes 25/75) yielded the crude title compound as colorless crystals (1.0g which is used in the next step.

### e) 3-[6-(2,4-Difluoro-phenylamino)-1.H.-pyrazolor3,4-.b.]pyridin-3-yl]-4-methoxy-.N.-pyridin-2-yl-benzamide

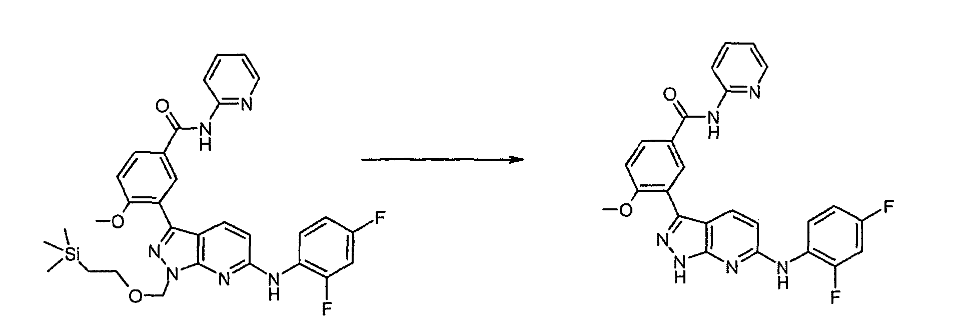

3-[6-(2,4-Difluoro-phenylamino)-1-(2-trimethylsilanyl-ethoxymethyl)-1.H.-pyrazolo[3,4-.b.]pyridin-3-yl]-4-methoxy-.N.-pyridin-2-yl-benzamide (1.0g; 1.66mol) in EtOH/HClconc (100ml; 1:1) is kept at room temperature for 15 minutes, poured on a saturated solution of Na₂CO₃ (300ml) and extracted with EtOAc/THF (10:1). The combined organic phases are washed with Na₂CO₃saturated and brine, evaporated to dryness and purified via chromatography (SiO2, acetone/hexanes 30/70 >100/0) to yield the title compound as white crystals (680mg; 86%).
1H-NMR (400MHz; DMSO): 3.95 (s, 3H); 6.82 (d, 1H); 7.08-7.18 (m, 2H); 7.29 (d, 1H); 7.35 (dt, 1H); 7.84 (dt, 1H); 7.91 (d, 1H); 8.13-8.23 (m, 3H); 8.30 (d, 1H); 8.40 (d, 1H); 9.01 (s, 1H, NH); 10.75 (s, 1H, NH); 13.20 (s, 1H, NH).
MS (m/z) ES+: 473 (MH+, 100).

### Scheme 4

3-Phenyl-1.H.-pyrazolo[3,4-.d.]pyrimidines of the type II" are prepared as follows:

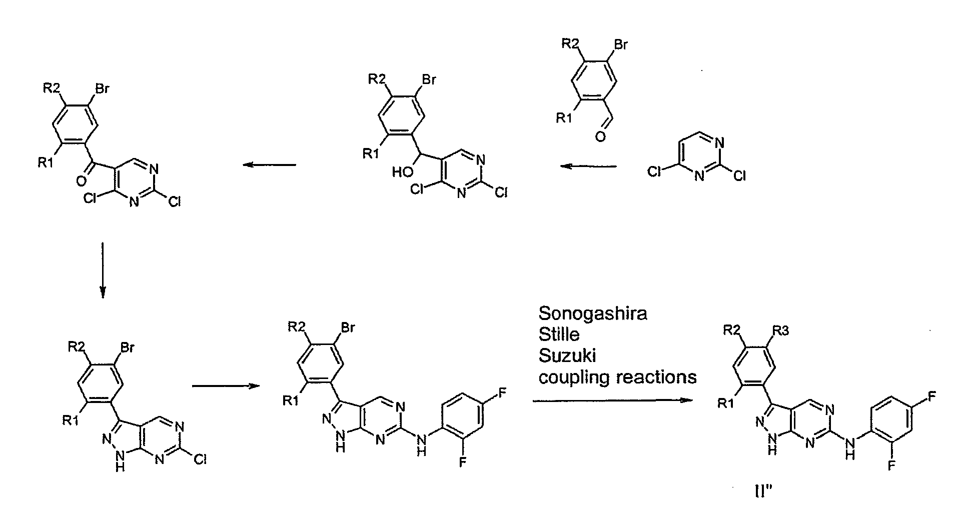

The synthesis of compounds of type II' is exemplified in example 24:

### Example 24: {3-[5-(3-Amino-3-methyl-but-1-ynyl)-2-methoxy-phenyl]-1.H.-pyrazolo[3,4-.d.]pyrimidin-6-yl}-(2,4-difluoro-phenyl)-amine

### a) (5-Bromo-2-methoxy-phenyl)-(2,4-dichloro-pyrimidin-5-yl)-methanol

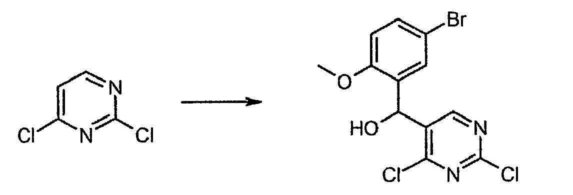

nBuLi (31.25ml; 50mmol of a 1.6M solution in hexanes; 50mmol) is added at -50C to a solution of diisopropylamine (7.7ml; 50mmol) in THF/isopentane (75ml/20ml), stirred for 10 minutes at -50C, then cooled to -100C. 2,4-Dichloropyrimidine (3.72g; 25mmol) in THF (45ml) is added and stirred at -100C for 15 minutes. 5-Bromo-o-anisaldehyde (3.22g; 15mmol) in THF (15ml) is added at -100C to the black reaction mixture, stirred for 10 minutes at -78C, poured on brine and extracted with TBME three times. The combined organic phases are dried over Na₂SO₄, evaporated and purified via chromatography (SiO₂, cyclohexane/TBME 7/3) to yield the title compound as yellow oil (2.2g; 40%), which is used in the next step.

### b) (5-Bromo-2-methoxy-phenyl)-(2,4-dichloro-pyrimidin-5-yl)-methanone

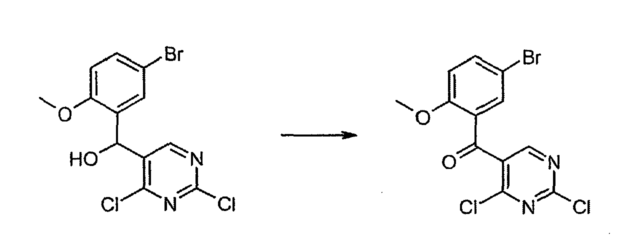

(5-Bromo-2-methoxy-phenyl)-(2,4-dichloro-pyrimidin-5-yl)-methanol (2.2g; 6mmol) is dissolved in acetone (190ml) and treated with 3 portions of MnO₂ (3x2.1g) added in 15 minutes intervals. The mixture was filtered, evaporated, recrystallised from TBME/hexanes to tield the title compound as yellow crystals (1.5g; 68%).
1H-NMR (400MHz; DMSO): 3.70 (s, 3H); 7.23 (d, 1H); 7.90 (m, 2H); 8.93 (s, 1H).
MS (m/z) EI: 362 (M+, 100); 327 (25); 215 (75); 213 (80).

### c) 3-(5-Bromo-2-methoxy-phenyl)-6-chloro-1.H.-pyrazolo[3,4-.d.]pyrimidine

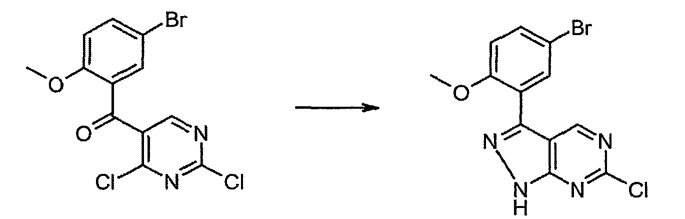

(5-Bromo-2-methoxy-phenyl)-(2,4-dichloro-pyrimidin-5-yl)-methanone (3.0g; 8.2mmol) is dissolved in warm EtOH (240ml) and cooled to 30C. A solution of H₂NNH₂.H₂O (3.26ml; 18.2mmol of a 24% solution in water) in 2N HCl (10.8ml; 21.75mmol) is added at 30C under stirring. NaHCO₃ (49.4ml; 57.2mmol; saturated solution) is added, the resulting precipitate stirred for 15 minutes, the reaction mixture poured on water and extracted with TBME twice. The combined organic phases are washed with brine, dried over Na₂SO₄ and evaporated to dryness to yield the title compound as a solid, which is purified via recrystallisation from TBME (2.2g; 75%)
1H-NMR (400MHz; DMSO): 3.92 (s, 3H); 7.25 (d, 1H); 7.68 (dd, 1H); 7.88 (d, 1H); 9.28 (s, 1H); 14.50 (s, 1H, NH).
MS (m/z) ES-: 339 (MH-, 100); 249 (50).

### d) [3-(5-Bromo-2-methoxy-phenyl)-1H.-pyazolo[3,4-.d.]pyrmidin-6-yl]-(2,4-difluoro-phepyl)-amine

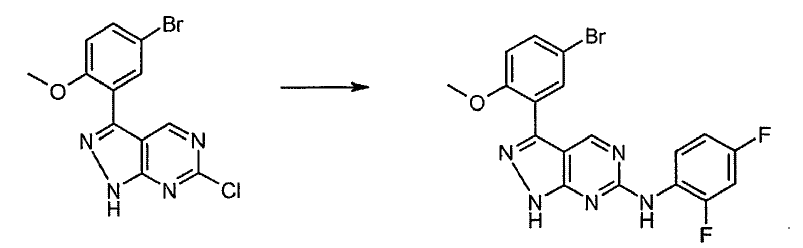

3-(5-Bromo-2-methoxy-phenyl)-6-chloro-1.H.-pyrazolo[3,4-.d.]pyrimidine (8.6g; 25mmol) and 2,4-difluoroaniline (25ml) are heated to 150C. After 5 minutes a precipitation is formed; heating is continued for another 10 minutes. The reaction mixture is cooled and diluted with hexanes (800ml), filtered, and the solid washed and triturated with water. The greenish product is dissolved in 1.5 liters of hot EtOAc/THF/EtOH (1:1:1) and concentrated to ∼100ml of volume. The resulting precipitate is diluted with TBME/hexanes (100ml/200ml) and filtered to yield the title compound as greenish crystals (9.7g; 89%).
1H-NMR (400MHz; DMSO): 3.91 (s, 3H); 7.12 (bt, 1H); 7.20 (d, 1H); 7.35 (bt, 1H); 7.63 (dd, 1H); 7.73 (m, 1H); 7.81 (d, 1H); 8.97 (s, 1H); 9.38 (s, 1H, NH); 13.50 (s, 1H, NH).
MS (m/z) ES-: 432, 430 (MH-; 100).

### e) {3-[5-(3-Amino-3-methyl-but-1-ynyl)-2-methoxy-phenyl]-1.H.-pyrazolo[3.4-.d.]pyimidin-6-yl}-(2,4-difluoro-phenyl)-amine

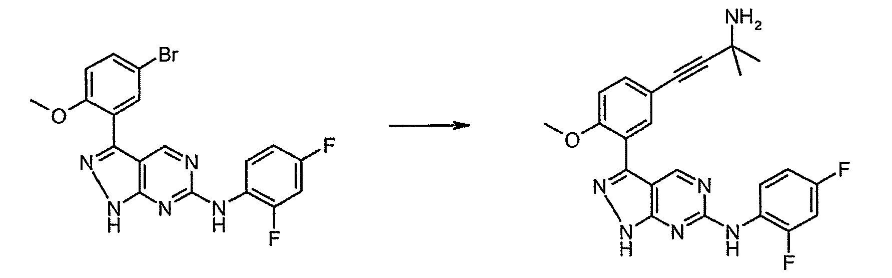

[3-(5-Bromo-2-methoxy-phenyl)-1.H.-pyrazolo[3,4-.d.]pyrimidin-6-yl]-(2,4-difluoro-phenyl)-amine (648mg; 1.5mmol) and 1,1-dimethyl-prop-2-ynylamine (1.5ml; 15mmol), PdCl₂(PPh₃)₂ (105mg; 0.15mmol), CuI (114mg; 0.6mmol), Cs₂CO₃ (487mg; 1.5mmol) are dissolved in Huenigs base (150ml) and heated to 130C for 1h. The reaction mixture is evaporated, taken up in TBME/EtOH, filtered and the filtrate purified via chromatography (SiO₂, TBME/MeOH 95/5 > TBME/MeOH/NH₃conc 90/9/1) to yield the title compound as yellow-brown crystals (100mg; 15%).
1H-NMR (400MHz; DMSO): 1.40 (s, 6H); 2.14 (bs, 2H, NH2); 3.90 (s, 3H); 7.11 (bt, 1H); 7.19 (d, 1H); 7.33 (bt, 1H); 7.42 (d, 1H); 7.66 (s, 1H); 7.72 (m, 1H); 8.94 (s, 1H); 9.23 (s, 1H); 13.45 (s, 1H, NH).
MS (m/z) ES-: 433 (M-H; 100).

### Example 25: 4-{3-[6-(2,4-Difluoro-phenylamino)-1.H.-pyrazolo[3,4-.d.]pyrimidin-3-yl]-4-methoxy-phenyl}-2-methyl-but-3-yn-2-ol

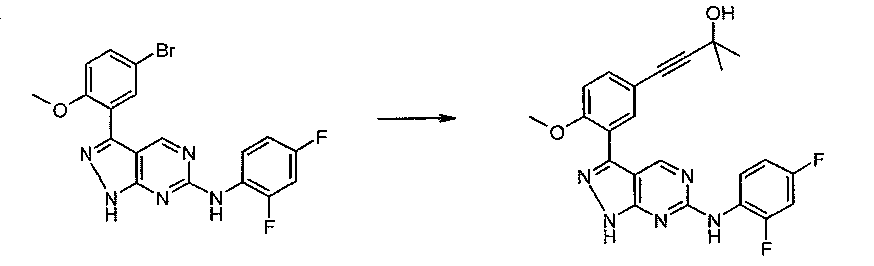

[3-(5-Bromo-2-methoxy-phenyl)-1.H.-pyrazolo[3,4-.d.]pyrimidin-6-yl]-(2,4-difluoro-phenyl)-amine (3.04g; 7mmol), 2-methyl-but-3-yn-2-ol (2.83ml; 28mmol), Et3N (16ml), DMF (28ml), PdCl₂(PPh₃)₂ (981mg; 40mmol) and CuI (266mg; 1.4mmol) are heated to 100C for 1 hour. The reaction mixture is evaporated, taken up in toluene (100ml), filtered and the filtrate purified by chromatography (SiO₂, toluene/TBME 70/30 > toluene/TBME 50/50) to yield the title compound as yellow solid, which is recrystallised from toluene/hexanes/TBME (1.6g; 52%).
1H-NMR (400MHz; DMSO): 1.49 (s, 6H); 3.93 (s, 3H); 5.43 (s, 1H, OH); 7.12 (dt, 1H); 7.21 (d, 1H); 7.35 (dt, 1H); 7.49 (dd, 1H); 7.71 (d, 1H); 7.72-7.78 (m, 1H); 8.97 (s, 1H); 9.23 (s, 1H,
NH); 13.35 (s, 1H, NH).
MS (m/z) ES+: 436 (MH+; 100).

### Example 26: 4-{3-[6-(2,4-Difluoro-phenylamino)-1.H.-prazolo[3,4-,d]pyrimidin-3-y]-4-methoxy-henyl}-2-methyl-butan-2-ol

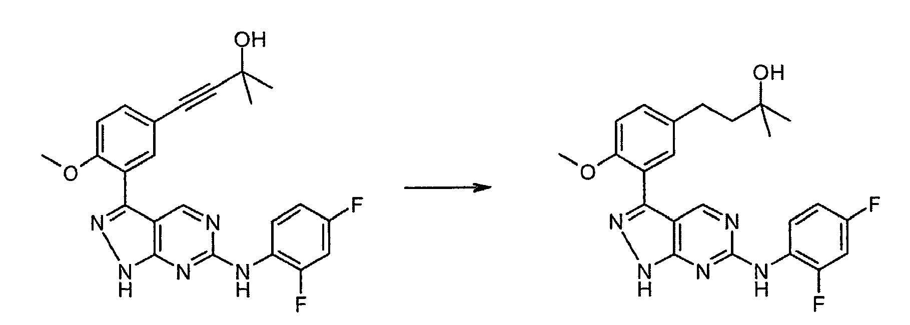

4- {3-[6-(2,4-Difluoro-phenylamino)-1,H.-pyrazolo[3,4-.d.]pyrimidin-3-yl]-4-methoxy-phenyl}-2-methyl-but-3-yn-2-ol (1.0g; 2.3mmol) is dissolved in EtOH (150ml) and hydrogenated under normal pressure over Pd/C (10%; 400mg) for 4 hours. The reaction is filtered, evaporated and purified via chromatography (SiO₂, acetone/hexanes 2/8) to yield the title compound as white solid, which is crystallised from acetone/TBME (700mg; 70%).
1H-NMR (400MHz; DMSO): 1.17 (s, 6H); 1.62-1.70 (m, 2H); 2.61-2.68 (m, 2H); 3.86 (s, 3H); 4.25 (s, 1H, OH), 7.10-7.15 (m, 2H); 7.28 (dd, 1H); 7.33 (dt, 1H); 7.52 (d, 1H); 7.71-7.78 (m, 1H); 8.93 (s, 1H); 9.20 (s, 1H, NH); 13.30 (s, 1H, NH).
MS (m/z) ES+: 440 (MH+; 100).

### Example 27: (2,4-Difluoro-phenyl)-{3-[5-(3-dimethylamino-prop-1-ynyl)-2-methoxy-phenyl]-1.H.-pyrazolo[3,4-.d.]pyrimidin-6-yl}-amine

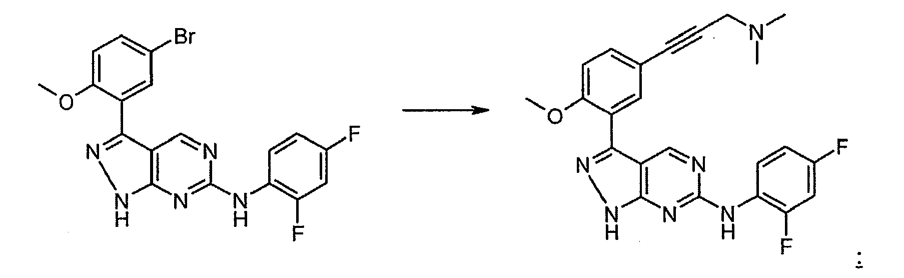

[3-(5-Bromo-2-methoxy-phenyl)-1.H.-pyrazolo[3,4-.d.]pyrimidin-6-yl]-(2,4-difluoro-phenyl)-amine (1g; 2.3mmol), R-BINAP (100mg; 1.6mmol), CuI (75mg; 0.39mmol), Pd(OAc)₂ (100mg; 0.44mmol), PPh₃ (175mg; 0.66mmol), NaOtBu (444mg; 4.6mmol) and dimethyl-prop-2-ynyl-amine (5.3ml; 46mmol) are dissolved in diethyleneglycol dimethylether (100ml) and heated in an autoclave at 135C for 1 hour. The reaction mixture is evaporated, dissolved in 2N HCl, washed with EtOAc, the aqueous phase alkalised with Na₂CO₃conc, filtered and extracted with EtOAc three times. The combined organic phases are dried over Na₂SO₄, evaporated to dryness and purified via chromatography (SiO₂, acetone/hexanes 4/6 > 100/0 > TBME/MeOH/NH₃conc 90/10/1) to yield the pure title compound after recrystallisation from methylene chloride (447mg; 45%).
1H-NMR (400MHz; DMSO): 2.24 (s, 6H); 3.44 (s, 2H); 3.90 (s, 3H); 7.10 (dt, 1H); 7.21 (d, 1H); 7.32 (dt, 1H); 7.70 (d, 1H); 7.50 (dd, 1H); 7.74 (m, 1H); 8.92 (s, 1H); 9.21 (s, 1H, NH); 13.45 (bs, 1H, NH).
MS (m/z) ES+: 435 (MH+, 100).

### Example 28: (2,4-Difluoro-phenyl)-{3-[2-methoxy-5-(3-morpholin-4-yl-prop-1-ynyl)-phenyl]-1.H.-pylazolo[3,4-.d.]primidin-6-yl}-amine

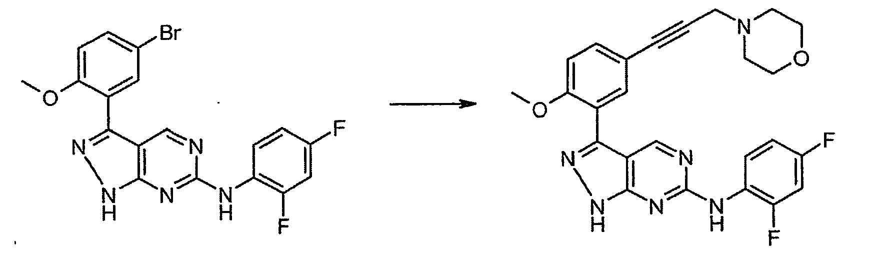

The title compounds is obtained from [3-(5-bromo-2-methoxy-phenyl)-1.H.-pyrazolo[3,4-.d.]pyrimidin-6-yl]-(2,4-difluoro-phenyl)-amine and 4-prop-2-ynyl-morpholine in analogy to example 27 in 70% yield as colorless crystals. 1H-NMR (400MHz; DMSO): 2.53 (bt, 4H); 3.50 (s, 2H); 3.61 (bt, 4H); 3.91 (s, 3H); 7.11 (dt, 1H); 7.22 (d, 1H); 7.32 (dt, 1H); 7.51 (dd, 1H); 7.71 (dd, 2H); 8.93 (s, 1H); 9.23 (s, 1H, NH); 13.45 (s, 1H, NH).
MS (m/z) ES+: 477 (MH+, 40), 390 (100).

### Example 29: (2,4-Difluoro-phenyl)-(3-{2-methoxy-5-r3-(4-methyl-pipin-1-yl)-prop-1-ynyl]-phenyl}-1.H.-pyrazolo[3,4-.d.]pyrimidin-6-yl)-amine

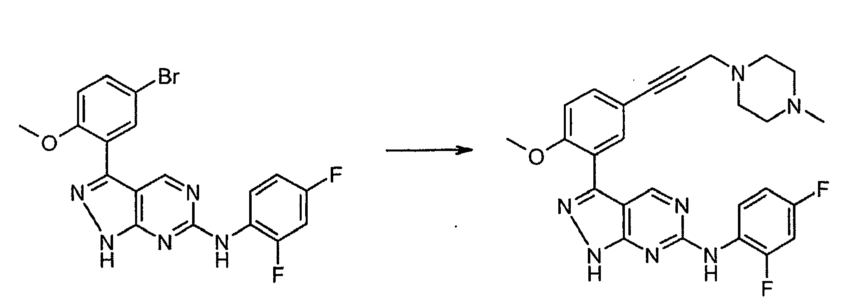

The title compound is obtained from [3-(5-bromo-2-methoxy-phenyl)-1.H.-pyrazolo[3,4-.d.]pyrimidin-6-yl]-(2,4-difluoro-phenyl)-amine and 1-methyl-4-prop-2-ynyl-piperazine in analogy to example 27 in 75% yield as colorless crystals.
1H-NMR (400MHz; DMSO): 2.15 (s, 3H); 2.34 (bs, 4H); 2.53 (bs, 4H); 3.51 (s, 2H); 3.90 (s, 3H); 7.10 (dt, 1H); 7.21 (d, 1H); 7.32 (dt, 1H); 7.49 (dd, 1H); 7.70 (d, 1H); 7.72 (m, 1H); 8.93 (s, 1H, NH); 9.22 (s, 1H, NH); 13.45 (bs, 1H, NH).
MS (m/z) ES+: 490 (MH+, 100).

### Example 30: 4- 3-[6-(2 4-Difluoro-phenylamino)-1.H.-pyrazolo[3,4-.d.]pyrimidin-3-yl]-4-methoxy-phenylethynyl}-1-methyl-piperidin-4-ol

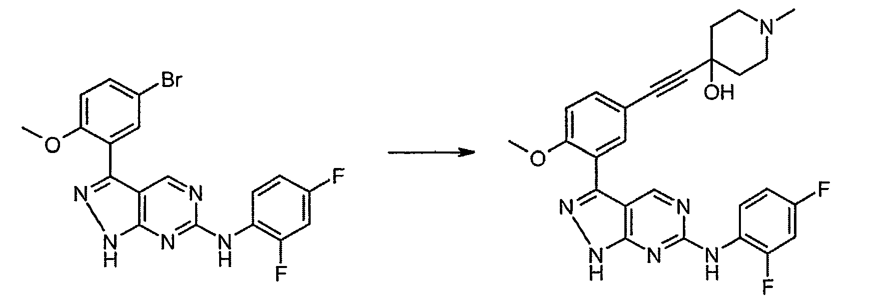

[3-(5-Bromo-2-methoxy-phenyl)-1.H.-pyrazolo[3,4-.d.]pyrimidin-6-yl]-(2,4-difluoro-phenyl)-amine (1.38g; 3.2mmol), 4-ethynyl-1-methyl-piperidin-4-ol (0.9g; 6.5mmol), PdCl₂(PPh₃)₂ (0.43g; 0.6mmol), Cs₂CO₃ (2.7g; 8.3mmol) and CuI (133mg; 0.7mmol) are dissolved in diethylene glycol dimethyl ether (32ml) and N,N-diisopropylethylamine (16ml) and heated to 140°C for 15 minutes. The reaction mixture is filtered, evaporated and purified via chromatography (SiO₂, EtOAc/MeOH/NH₃conc 95/4.5/0.5 > 90/9/1) to yield the title compound, which is crystallised from EtOH/TBME (900mg; 54%).
1H-NMR (400MHz; DMSO): 1.72 (m, 2H); 1.83 (m, 2H); 2.16 (s, 3H); 2.26 (m, 2H); 2.54 (m, 2H); 3.91 (s, 3H); 5.50 (s, 1H, OH); 7.11 (dt, 1H); 7.22 (d, 1H); 7.32 (dt, 1H); 7.48 (dd, 1H);
7.69 (d, 1H); 7.72 (m, 1H); 8.93 (s, 1H); 9.24 (s, 1H, NH); 13.44 (bs, 1H, NH)
MS (m/z) ES+: 491 (MH+, 100).

### Example 31: (2,4-Difluoro-phenyl)-{3-[5-((E)-3-dimethylamino-propenyl)-2-methoxy-phenyl]-1.H.-pyrazolo[3,4-.d.]pyrimidin-6-yl}-amine

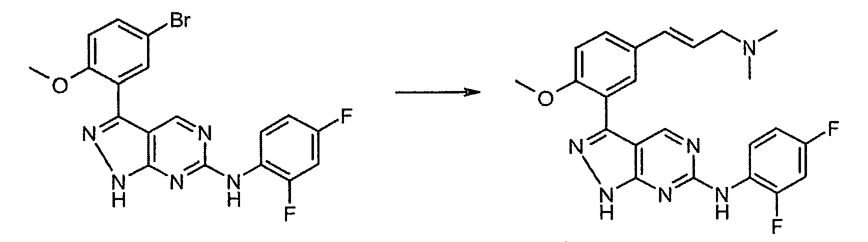

Dimethyl-((E)-3-tributylstannanyl-allyl)-amine (312mg, 0.83mmol) and [3-(5-bromo-2-methoxy-phenyl)-1.H.-pyrazolo[3,4-.d.]pyrimidin-6-yl]-(2,4-difluoro-phenyl)-amine (200mg, 0.69mmol) are added to a solution of Pd(OAc)₂ (15mg, 0.07mmol) and PPh₃ (75mg, 0.2mmol) in diethylene glycol dimethyl ether (16ml) and heated to 130C under argon for 40 minutes. The reaction mixture is evaporated to dryness and purified via a first chromatography (SiO₂ acetone/hexanes 6/4 > 9/1 > acetone/MeOH 9/1) followed by a second chromatography (SiO₂, TBME/MeOH/NH₃conc 95/5/0.5 > 90/10/1) to yield the title compound as pale yellow crystals (60mg; 20%).
1H-NMR (400MHz; DMSO): 2.16 (s, 6H); 3.01 (bd, 2H); 3.88 (s, 3H); 6.19 (dt, 1H); 6.52 (d, 1H); 7.10 (bt, 1H); 7.17 (d, 1H); 7.32 (bt, 1H); 7.52 (bd, 1H); 7.71-7.80 (m, 2H); 8.91 (s, 1H); 9.20 (bs, 1H, NH); 13.40 (s, 1H, NH).
MS (m/z) ES+: 424 (MH+, 100).

### Example 32: (2 4-Difluoro-phenyl)-{3-[2-methoxy-5-((E)-3-morpholin-4-yl-propenyl)-phenyl]-1,H.-pyrazolo[3,4-.d.]pyrimidin-6-yl}-amine

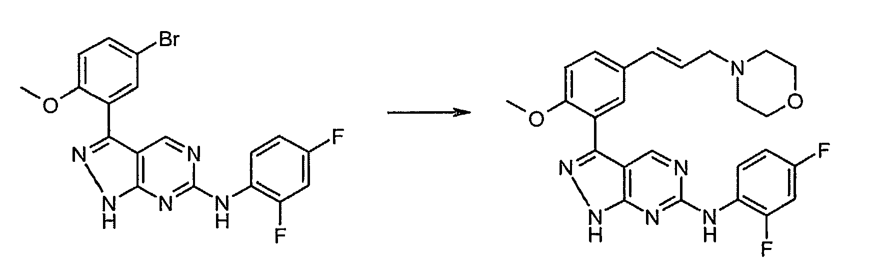

The title compound is obtained from 4-((E)-3-tributylstannanyl-allyl)-morpholine and [3-(5-bromo-2-methoxy-phenyl)-1.H.-pyrazolo[3,4-.d.]pyrimidin-6-yl]-(2,4-difluoro-phenyl)-amine as colorless crystals in analogy to example 30 in 47% yield.
1H-NMR (400MHz; DMSO): 2.39 (bs, 4H); 3.07 (bd, 2H); 3.58 (bt, 4H); 3.88 (s, 3H); 6.21 (dt, 1H); 6.54 (d, 1H); 7.11 (dt, 1H); 7.17 (d, 1H); 7.33 (dt, 1H); 7.55 (dd, 1H); 7.71 (m, 2H); 8.91 (s, 1H); 9.21 (s, 1H, NH); 13.40 (s, 1H, NH).
MS (m/z) ES+: 479 (MH+, 100).

### Example 33: (2,4-Difluoro-pheyl)-(3-{2-methoxy-5-[(E)-3-(4-methyl-piperazin-1-yl)-propenyl]-phenyl}-1.H.-pyrazolo[3,4-.d.]pyrimidin-6-yl)-amine

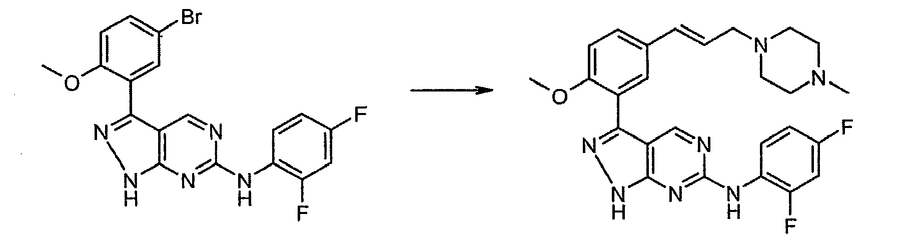

The title compound is obtained from 1-methyl-4-((E)-3-tributylstannanyl-allyl)-piperazine and [3-(5-bromo-2-methoxy-phenyl)-1.H.-pyrazolo[3,4-.d.]pyrimidin-6-yl]-(2,4-difluoro-phenyl)-amine as colorless crystals in analogy to example 31 in 10% yield.
1H-NMR (400MHz; DMSO: 2.14 (s, 3H); 2.25-2.50 (m, 8H); 3.07 (d, 2H); 3.87 (s, 3H); 6.18 (dt, 1H); 6.52 (d, 1H); 7.11 (dt, 1H); 7.17 (d, 1H); 7.32 (dt, 1H); 7.52 (dd, 1H); 7.70-7.78 (m, 2H); 8.91 (s, 1H); 9.22 (s, 1H, NH); 13.40 (s, 1H, NH).
MS (m/z) ES+: 492 (MH+, 100).

### Scheme 5

The synthesis of phenyl-(3-phenyl-1.H.-pyrazolo[3,4-.b.]pyrazin-6-yl)-amines of type I"' 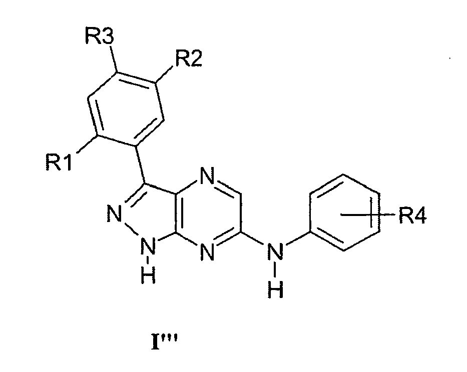 is exemplified in Examples 33 and 35:

### Example 34: 4-{3-[6-(2,4-Difluoro-phenylamino)-1.H.-pyrazolo[3,4-.b.]pyrazin-3-yl]-4-methoxy-phenyl}-2-methyl-but-3-yn-2-ol

### a) (5-Bromo-2-methoxy-phenyl)-(3,5-dichloro-pyrazin-2-yl)-methanol

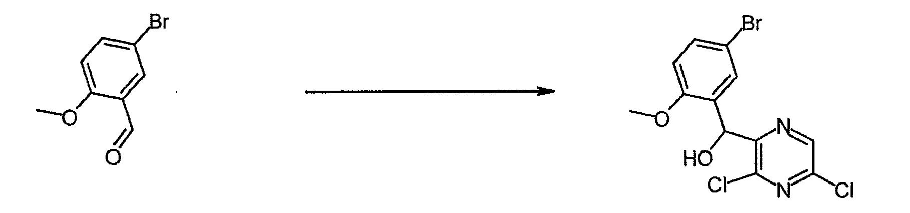

Tetramethylpiperidine (5.25ml; 30.75mmol) in THF (450ml) is cooled to -15C and treated with nBuLi (18.7ml; 30mmol of a 1.6M solution in hexanes) for 10 minutes and then cooled to -90C. 2,6-Dichloro-pyrazine (3.34g; 22.5mmol) in THF (45ml) is added within a minute and the red-brown reaction mixture stirred for 30 seconds at -90C to - 100C. 5-Bromo-2-methoxybenzaldehyde (3.2g; 15mmol) in THF (45ml) is added at -90C within one minute and the reaction stirred at -78C for 10 minutes. The black reaction mixture is poured on brine and extracted twice with TBME. The combined organic phases are washed with water, dried over Na₂SO₄, filtered and evaporated to dryness. Purification via chromatography (SiO₂ toluene) delivers the title compound as colorless crystals (3g; 55%).
1H-NMR (400MHz; DMSO: 3.62 (s, 3H); 6.25 (s, 1H); 6.90 (d, 1H); 7.45 (dd, 1H); 7.70 (d, 1H); 8.71 (s, 1H).
MS (m/z) EI: 364 (M+, 90); 333 (65); 215 (100).

### b) (5-Bromo-2-methoxy-phenyl)-(3,5-dichloro-pyrazin-2-yl)-methanone

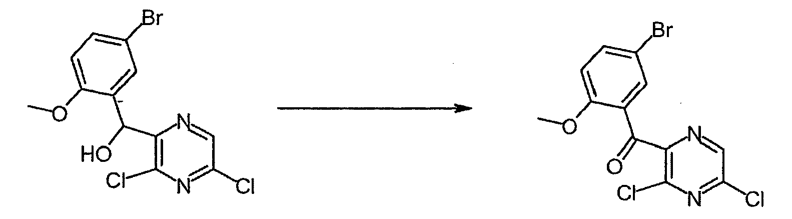

(5-Bromo-2-methoxy-phenyl)-(3,5-dichloro-pyrazin-2-yl)-methanol (9g; 24.7mmol) in acetone (800ml) is treated for 15 minutes at room temperature with Jones reagent (53ml; 123mmol of a 2.33mol solution). Acetone (∼700ml) is distilled off and the residue poured on saturated Na₂CO₃ and extracted with EtOAc twice. The combined organic phases are dried over Na₂SO₄, filtered and evaporated to dryness to yield the title compound as white crystals (7.9g; 88%).
1H-NMR (400MHz; DMSO): 3.62 (s, 3H); 6.71 (d, 1H); 7.83-7.93 (m, 2H); 8.90 (s, 1H).
MS (m/z) EI: 362 (M+, 30); 331 (30); 213 (100).

### c) 3-(5-Bromo-2-methoxy-phenyl)-6-chloro-1.H.-pyrazolo[3,4-.b.]pyrazine

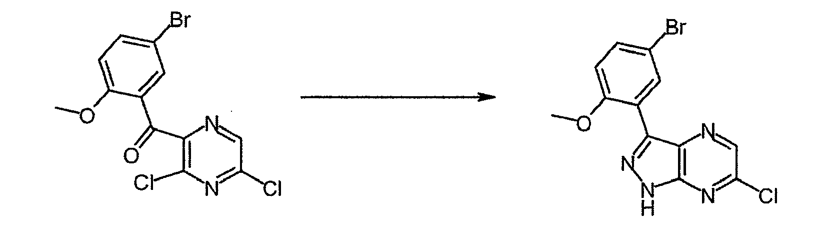

(5-Bromo-2-methoxy-phenyl)-(3,5-dichloro-pyrazin-2-yl)-methanone (7.9g; 21.8mmol) is dissolved in warm (50C) MeOH (790ml) and treated with H₂NNH₂.H₂O (2.37ml; 48mmol of a 98% solution in water). After 1 hour at 50C a second portion of H₂NNH₂.H₂O (1.2ml; 24mmol of a 98% solution in water) is added and stirring continued for 30 minutes. Filtration of the yellow solid delivers the title compound (2.66g; 36%).
1H-NMR (400MHz; DMSO): 3.83 (s, 3H); 6.70 (d, 1H); 7.65 (dd, 1H); 7.95 (bs, 1H); 8.75 (s,
1H).
MS (m/z) ES- 339 (MH-; 100).

### d) [3-(5-Bromo-2-methoxy-phenyl)-1H.-pyrazolo[3,4-.b]pyrazin-6-yl]-(2,4-difluoro-phenyl)-amine

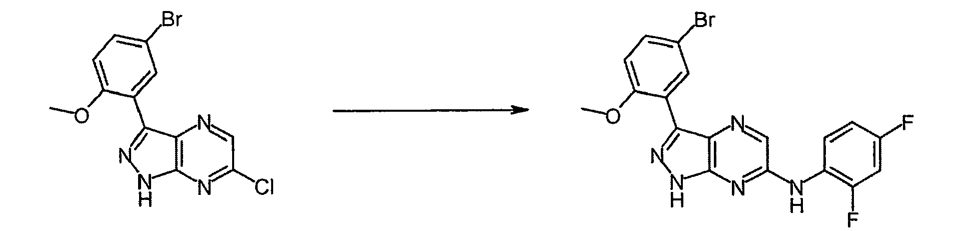

3-(5-Bromo-2-methoxy-phenyl)-6-chloro-1.H.-pyrazolo[3,4-.b.]pyrazine (2.8g; 8.27mmol) and 2,4-difluoroaniline (16.5ml) are heated at 175C for 1 hour. The reaction mixture - originally a suspension - forms a solution, then precipitates again. Hexane (500ml) is added to the cooled reaction, the precipitate filtered off, dissolved in EtOAc/THF (500ml 4:1) and washed with water (400ml). The aqueous phase is extracted with EtOAc, the combined organic phases dried over Na₂SO₄ and evaporated to dryness to yield the title compound, which is crystallised from EtOAc/TBME (3.3g; 91%).
1H-NMR (400MHz; DMSO): 3.81 (s, 3H); 7.13-7.22 (m, 2H); 7.41 (dt, 1H); 7.62 (dd, 1H); 7.85 (s, 1H); 8.08-8.18 (m, 1H); 8.47 (s, 1H); 9.61 (bs, 1H, NH); 13.50 (s, 1H, NH).
MS (m/z) ES-: 432 (M+, 100); 430 (100).

### d) 4-{3-[6-(2,4-Difluoro-phenylamino)-1.H.-pyrazolo[3,4-.b.]pyrazin-3-yl]-4-methoxy-phenyl}-2-methyl-but-3-yn-2-ol

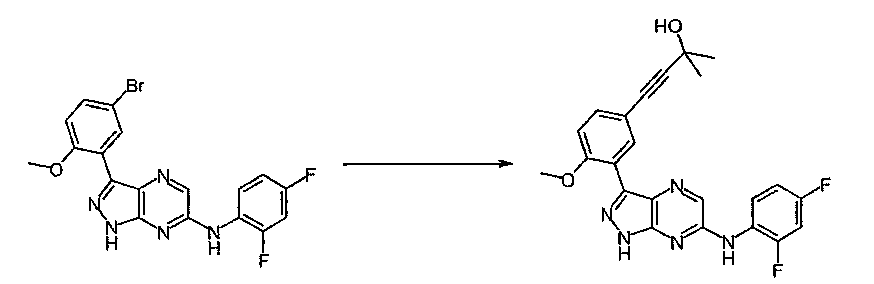

The title compound is obtained in 42% yield from [3-(5-bromo-2-methoxy-phenyl)-1.H.-pyrazolo[3,4-.b.]pyrazin-6-yl]-(2,4-difluoro-phenyl)-amine and 2-methyl-but-3-yn-2-ol in analogy to example 25.
1H-NMR (400MHz; DMSO): 1.48 (s, 6H); 3.84 (bs, 3H); 5.43 (s, 1H, OH); 7.11-7.19 (m, 2H); 7.38 (dt, 1H); 7.45 (d, 1H); 7.70-7.85 (bs, 1H); 8.17 (bs, 1H); 8.37 (s, 1H); 9.55 (bs, 1H, NH); 13.30 (s, 1H, NH).
MS (m/z) ES-: 434 (MH-; 100).

### Example 35: (2,4-Difluoro-phenyl)-{3-[5-(3-dimethylamino-prop-1-ynyl)-2-methoxy-phenyl]-1.H.-pyrazolo[3,4-.b.]pyrazin-6-yl}-amine

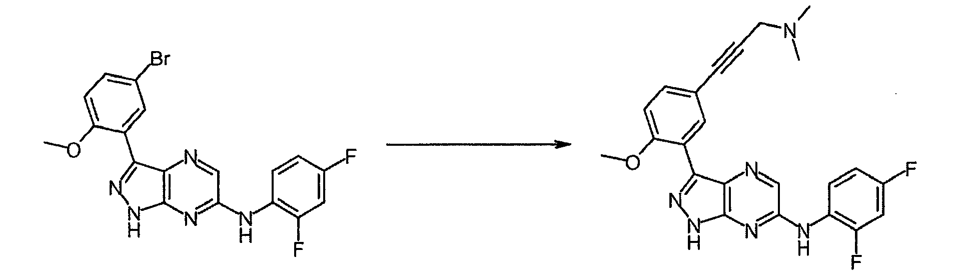

The title compound is obtained in 20% yield from [3-(5-bromo-2-methoxy-phenyl)-1.H.-pyrazolo[3,4-.b.]pyrazin-6-yl]-(2,4-difluoro-phenyl)-amine and dimethyl-prop-2-ynyl-amine in analogy to example 27.
1H-NMR (400MHz; DMSO): 2.27 (s, 6H); 3.47 (s, 2H); 3.82 (s, 3H); 7.19 (bd, 2H); 7.41 (t, 1H); 7.52 (d, 1H); 7.74 (bs, 1H); 7.78 (bs, 1H); 8.18 (bs, 1H); 9.60 (s, 1H, NH); 13.40 (s, 1H,
NH).
MS (m/z) ES-: 433 (MH-, 100).

The Agents of the Invention, as defined above, e.g., of formula I, II and V particularly as exemplified, in free or pharmaceutically acceptable acid addition salt form, exhibit pharmacological activity and are useful as pharmaceuticals, e.g. for therapy, in the treatment of diseases and conditions as hereinafter set forth.

In particular Agents of the Invention possess p38 MAP kinase (Mitogen Activated Protein Kinase) inhibiting activity. Thus the Agents of the Invention act to inhibit production of inflammatory cytokines, such as TNF and IL-1, and also to potentially block the effects of these cytokines on their target cells. These and other pharmacological activities of the Agents of the Invention as may be demonstrated in standard test methods for example as described below:

### p38 MAP kinase Assay

The substrate (GST-ATF-2; a fusion protein comprising amino acids 1-109 of ATF-2 and the GST protein obtained by expression in E. coli) is coated onto the wells of microtiter plates (50 µl/well; 1 µg/ml in PBS/0.02% Na azide) overnight at 4 °C. The following day, the microtiter plates are washed four times with PBS/0.5% Tween 20/0.02% Na azide and are blocked with PBS/2% BSA/0.02% Na Azide for 1 h at 37 °C. Plates are washed again 4 times with PBS/0.5% Tween 20/0.02% Na azide. The kinase cascade reaction is then started by adding the following reactants in 10 µl aliquots to a final reaction volume of 50 µl.
1. Agents of the Invention titrated from 10 to 0.001 µM in 10-fold dilutions or solvent (DMSO) or H₂O.
2. Kinase buffer (5x); pH 7.4; 125 mM Hepes (Stock at 1M; Gibco #15630-056), 125 mM β-glycerophosphate (Sigma #G-6251):125 mM MgCl₂ (Merck #5833); 0.5 mM Sodium orthovanadate (Sigma #5-6508), 10 mM DTT (Boehringer Mannheim #708992). The (5x) kinase buffer must be prepared fresh the day of the assay from 5x stock solutions kept at RT.
   DTT is kept at -20 °C and is added as the last reagent.
3. His-p38 MAP kinase (10 ng/well; Novartis - a fusion protein comprising full length murine p38 MAP kinase and a His tag, obtained by expression in E. coli)
4. cold ATP (final concentration 120 µM; Sigma #A-9187)
5. Water
   After 1h at 37 °C the kinase reaction is terminated by washing the plates four times as previously
   described. Phosphorylated GST-ATF-2 is then detected by adding:

1. the PhosphoPlus ATF-2 (Thr71) Antibody (50 µl/well; 1/1000 final dilution in PBS/2%
   BSA/0.02% Na Azide; New England Biolabs #9221L) for 90 min at RT.
2. Biotin labelled goat-anti-rabbit IgG (50 µl/well; 1/3000 final dilution in PBS/2% BSA/0.02%
   Na Azide; Sigma #B-9642) for 90 min at RT.
3. Streptavidin-alkaline phosphatase (50 µl/well; 1/5000 dilution in PBS/2% BSA/0.02% Na
   Azide; Jackson Immunoresearch #016-050-084) for 30 min at RT.
4. Substrate (100 µl/well; Sigma 104 Phosphatase substrate tablets, 5 mg/tablet; #104-105; 1 mg/ml in substrate buffer, Diethanolamine (97 ml/l; Merck #803116) + MgCl₂.6H₂O (100
   mg/l; Merck #5833) + Na Azide (0.2 g/l) + HCl 1M to pH 9.8) 30 min at RT.
   After step 1,2 and 3 the microtiter plates are washed four times with PBS/0.5% Tween 20/0.02% Na azide. After step 4, the plates are read in a Bio-Rad microplate reader in a dual wavelength mode (measurement filter 405 nm and reference filter 490 nm). The bachground value (without ATP) is subtracted and IC₅₀ values are calculated using the Origin computer program (4 parameter logistic function).

Agents of the Invention typically have IC₅₀s for p38 MAP kinase inhibition in the range from about 100 nM to about 10 nM or less when tested in the above assay.

### Assay for Inhibition of TNF-α release from hPBMCs

Human peripheral blood mononuclear cells (hPBMCs) are prepared from the peripheral blood of healthy volunteers using ficoll-hypaque density separation according to the method of Hansell et al., J. Imm. Methods (1991) 145: 105. and used at a concentration of 10⁵ cells/well in RPMI 1640 plus 10% FCS. Cells are incubated with serial dilutions of the test compounds for 30 minutes at 37°C prior to the addition of IFNg (100 U/ml) and LPS (5 mg/ ml) and subsequently further incubated for three hours. Incubation is terminated by centrifugation at 1400 RPM for 10 min. TNF-α in the supernatant is measured using a commercial ELISA (Innotest hTNFa, available from Innogenetics N.V., Zwijnaarde, Belgium). Agents of the Invention are tested at concentrations of from 0 to 10 mM. Exemplified Agents of the Ivention typically suppress TNF release in this assay with an IC₅₀ of from about 10 mM to about 100 nM or less when tested in this assay.

### Assay for Inhibition of TNF-α Production in LPS stimulated mice

Injection of lipopolysaccharide (LPS) induces a rapid release of soluble tumour necrosis factor (TNF-α) into the periphery. This model is be used to analyse prospective blockers of TNF release in vivo.

LPS (20 mg/kg) is injected i.v. into OF1 mice (female, 8 week old). One (1) hour later blood is withdrawn from the animals and TNF levels are analysed in the plasma by an ELISA method using an antibody to TNF-α. Using 20 mg/kg of LPS levels of up to 15 ng of TNF-α / ml plasma are usually induced. Compounds to be evaluated are given either orally or s.c. 1 to 4 hours prior to the LPS injection. Inhibition of LPS-induced TNF-release is taken as the readout.

Agents of the Invention typically inhibit TNF production to the extent of up to about 50% or more in the above assay when administered at 10 mg/kg p.o. and up to about 98% or more when administered at 30 mg/kg p.o..

As indicated in the above assays Agents of the Invention are potent inhibitors of TNF-α release. Accordingly, the Novel Compounds have pharmaceutical utility as follows:

Agents of the Invention are useful for the prophylaxis and treatment of diseases or pathological conditions mediated by cytokines such as TNFα and IL-1, e.g., inflammatory conditions, autoimmune diseases, severe infections, and organ or tissue transplant rejection, e.g. for the treatment of recipients of heart, lung, combined heart-lung, liver, kidney, pancreatic, skin or corneal transplants and for the prevention of graft-versus-host disease, such as following bone marrow transplants.

Agents of the Invention are particularly useful for the treatment, prevention, or amelioration of autoimmune disease and of inflammatory conditions, in particular inflammatory conditions with an aetiology including an autoimmune component such as arthritis (for example rheumatoid arthritis, arthritis chronica progrediente and arthritis deformans) and rheumatic diseases. Specific auto-immune diseases for which Agents of the Invention may be employed include autoimmune haematological disorders (including e.g. hemolytic anaemia, aplastic anaemia, pure red cell anaemia and idiopathic thrombocytopenia), systemic lupus erythematosus, polychondritis, sclerodoma, Wegener granulamatosis, dermatomyositis, chronic active hepatitis, myasthenia gravis, psoriasis, Steven-Johnson syndrome, idiopathic sprue, autoimmune inflammatory bowel disease (including e.g. ulcerative colitis and Crohn's disease), endocrine ophthalmopathy, Graves disease, sarcoidosis, multiple sclerosis, primary biliary cirrhosis, juvenile diabetes (diabetes mellitus type I), uveitis (anterior and posterior), keratoconjunctivitis sicca and vernal keratoconjunctivitis, interstitial lung fibrosis, psoriatic arthritis and glomerulonephritis (with and without nephrotic syndrome, e.g. including idiopathic nephrotic syndrome or minimal change nephropathy).

Agents of the Invention are also useful for the treatment, prevention, or amelioration of asthma, bronchitis, pneumoconiosis, pulmonary emphysema, and other obstructive or inflammatory diseases of the airways.

Agents of the Invention are useful for treating undesirable acute and hyperacute inflammatory reactions which are mediated by TNF, especially by TNFα, e.g., acute infections, for example septic shock (e.g., endotoxic shock and adult respiratory distress syndrome), meningitis, pneumonia; and severe burns; and for the treatment of cachexia or wasting syndrome associated with morbid TNF release, consequent to infection, cancer, or organ dysfunction, especially AIDS -related cachexia, e.g., associated with or consequential to HIV infection.

Agents of the Invention are also useful for the treatment of neurodegenerative diseases, such as Alzheimer's disease, acute encephalitis, brain injury, multiple sclerosis including demyelation and oligiodendrocyte loss in multiple sclerosis and inflammatory nervous system diseases, such as neuroinflammatory and stroke.

Agents of the Invention are particularly useful for treating diseases of bone metabolism including osteoarthritis, osteoporosis and other inflammatory arthritides.

For the above indications the appropriate dosage will, of course, vary depending, for example, on the particular Agent of the Invention employed, the subject to be treated, the mode of administration and the nature and severity of the condition being treated. However, in general, satisfactory results in animals are obtained at daily dosages of from about 1 to about 10mg/kg/day p.o.. In larger mammals, for example humans, an indicated daily dosage is in the range of from about 50 to about 750mg of an Agent of the Invention administered orally once or, more suitably, in divided dosages two to four times/day.

The Agents of the Invention may be administered by any conventional route, e.g. orally, for example in the form of solutions for drinking, tablets or capsules or parenterally, for example in the form of injectable solutions or suspensions. Normally for systemic administration oral dosage forms are preferred, although for some indications the Agents of the Invention may also be administered topically or dermally, e.g. in the form of a dermal cream or gel or like preparation or, for the purposes of application to the eye, in the form of an ocular cream, gel or eye-drop preparation; or may be administered by inhalation, e.g., for treating asthma. Suitable unit dosage forms for oral administration comprise e.g. from 25 to 250mg of Agent of the Invention per unit dosage.

In accordance with the foregoing the present invention also provides in a further series of embodiments:
A. A medicament for inhibiting production of soluble TNF, especially TNFα, or of reducing inflammation in a subject (i.e., a mammal, especially a human) in need of such treatment which method comprises administering to said subject an effective amount of an Agent of the Invention, or a method of treating any of the above mentioned conditions, particularly a method of treating an inflammatory or autoimmune disease or condition, e.g. rheumatoid arthritis, or alleviating one or more symptoms of any of the above mentioned conditions.
B. An Agent of the Invention for use as a pharmaceutical, e.g. for use as an immunosuppressant or antiinflammatory agent or for use in the prevention, amelioration or treatment of any disease or condition as described above, e.g., an autoimmune or inflammatory disease or condition.
C. A pharmaceutical composition comprising an Agent of the Invention in association with a pharmaceutically acceptable diluent or carrier, e.g., for use as an immunosuppressant or anti-inflammatory agent or for use in the prevention, amelioration or treatment of any disease or condition as described above, e.g., an autoimmune or inflammatory disease or condition.
D. Use of an Agent of the Invention in the manufacture of a medicament for use as an immunosuppressant or anti-inflammatory agent or for use in the prevention, amelioration or treatment of any disease or condition as described above, e.g., an autoimmune of inflammatory disease or condition.

## Claims

1. A compound of formula I 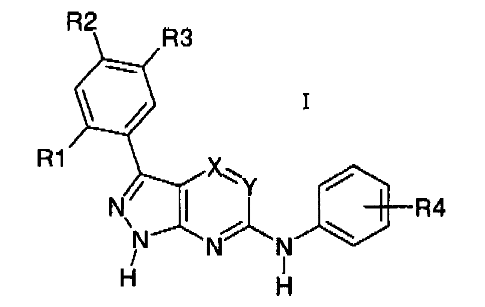 wherein
X and Y are independently carbon or nitrogen,
R1 is H, halogen, hydroxy, C₁-C₇alkoxy, C₁-C₇alkyl or halo-substituted C₁-C₇alkyl;
R2 is H, or optionally substituted (heterocyclyl, C₁-C₇alkyl, C₂-C₇alkenyl, C₂-C₇alkynyl or
C₁-C₇alkoxy);
R3 is H, or optionally substituted (heterocyclyl, C₁-C₇alkyl, C₂-C₇alkenyl, C₂-C₇alkynyl or C₁-C₇alkoxy); or
R2 and R3 are linked together to form a 4- to 6-membered heterocyclic ring containing one or more hetero atoms selected from O, S, N or NR, where R is H or C₁-C₇alkyl;
R4 represents one, two or three halogen substituents which may be the same or different, or a pharmaceutically-acceptable and -cleavable ester or acid addition salt thereof,
with the exception of [3-(2-chlorophenyl)-1H-pyrazolo[3,4-d]pyrimidin-6-yl]-(2-fluorophenyl]-amine, [3-(2-chlorophenyl)-1H-pyrazolo[3,4-b]pyrazin-6-yl]-(2,4-difluorophenyl)-amine and [3-(2-chlorophenyl)-1H-pyrazolo[3,4-d]pyrimidin-6-yl]-(2,4-difluorophenyl)-amine.

2. A compound according to claim 1 of formulae I'. I"or I"' 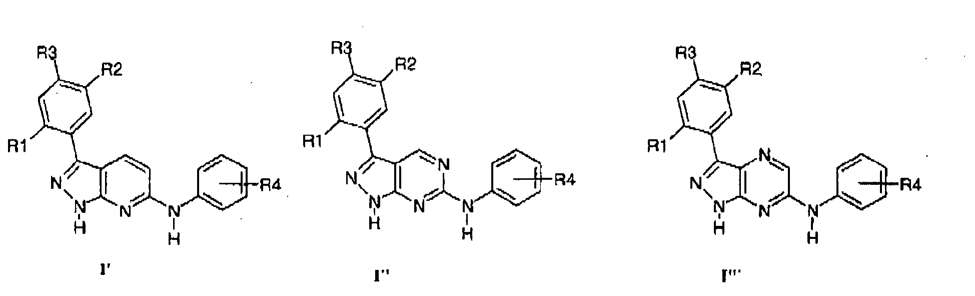 wherein the substituents R1. R2. R3 and R4 are as defined in claim 1,
or a pharmaceutically-acceptable and -cleavable ester or acid addition salt thereof,
with the exception of [3-(2-chlorophenyl)-1H-pyrazolo[3,4-d]pyrimidin-6-yl]-(2-fluorophenyl]-amine, [3-(2-chlorophenyl)-1H-pyrazolo[3,4-b]pyrazin-6-yl]-(2,4-difluorophenyl)-amine and [3-(2-chlorophenyl)-1H-pyrazolo[3,4-d]pyrimidin-6-yl]-(2,4-difluorophenyl)-amine.

3. A compound of the formula I according to claim 1, wherein R4 is 2,4-difluoro and X, Y, R1, R2 and R3 are as defined in claim 1, or a pharmaceutically acceptable and cleavable ester or acid addition salt thereof, with the exception of [3-(2-chlorophenyl)-1H-pyrazolo[3,4-b]pyrazin-6-yl]-(2,4-difluoro-phenyl)-amine and [3-(2-chlorophenyl)-1H-pyrazolo[3,4-d]pyrimidin-6-yl]-(2,4-difluoro-phenyl)-amine.

4. A compound according to claim 1 of Formulae II 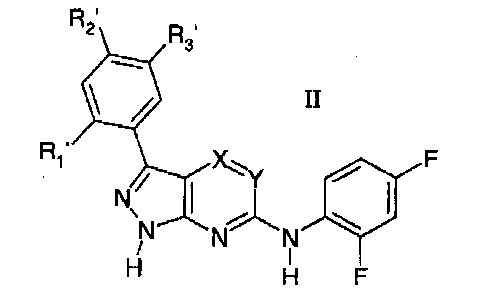 wherein
X and Y are independently carbon or nitrogen,
R1' is halo, C₁-C₄alkyl, C₁-C₄alkoxy or halo-substituted C₁-C₄alkyl;
R2' and R3' are, independently of one another, H or optionally substituted (C₁-C₄alkyl, C₁-C₄ alkoxy, C₂-C₇alkenyl, C₂-C₇alkynyl, C₅-C₇N-heterocyclyl, C₅-C₇heterocyclylC₁-C₄alkoxy, C₅-C₇heterocyclylC₁-C₄alkyl, C₅-C₇heterocyclylC₂-C₄alkenyl, C₅-C₇heterocyclylC₂-C₄alkynyl) wherein the C₅-C₇heterocyclyl optionally contains a second hetero atom and the optional substitution comprises 1 or 2 substituents, separately selected from C₁-C₄ alkyl, halogen, hydroxy, C₁-C₄alkoxy, or optionally mono-or di-N-C₁₋₄alkyl substituted amino or R2' and R3' are linked by a methylenedioxy linker or are linked in an imidazole ring optionally substituted by 1 or 2 substituents, separately selected from C₁₋₄alkyl, halogen, hydroxy. C₁₋₄alkoxy, and optionally mono-or di-N-C₁₋₄alkyl substituted amino; or a pharmaceutically-acceptable and -cleavable ester or acid addition salt thereof,
with the exception of [3-(2-chlorophenyl)-1H-pyrazolo[3,4-b]pyrazin-6-yl]-(2,4-difluorophenyl)-amine and [3-(2-chlorophenyl)-1H-pyrazolo[3,4-d]pyrimidin-6-yl]-(2,4-difluorophenyl)-amine.

5. A compound of formula I according to claim 1 selected from:
(2,4-Difluoro-phenyl)-[3-(2-methoxy-phenyl)-1.H.-pyrazolo[3,4-,b,]pyridin-6-yl]-amine; [3-(6-Chloro-benzo[1,3]dioxol-5-yl)-1.H.-pyrazolo[3,4-.b.]pyridin-6-yl]-(2,4-difluorophenyl)-amine;
[3-(2-Chloro-phenyl)-1.H.-pyrazolo[3,4-.b.]pyridin-6-yl]-(2,4-difluoro-phenyl)-amine;
(2,4-Difluoro-phenyl)-[3-(2-trifluoromethyl-phenyl)-1.H.-pyrazolo[3,4-.b.]pyridin-6-yl]-amine;
(2,4-Diftuoro-phenyl)-[3-(2,4-dimethoxy-phenyl)-1.H.-pyrazolo[3,4-.b.]pyridin-6-yl]-amine;
[3-(2-Chloro-4,5-dimethoxy-phcnyl)-1H.-pyrazolo[3.4-.b.]pyridin-6-yl]-(2,4-difluorophenyl)-amine;
(2,4-Difluoro-phenyl)-[3-(2-methoxy-5-morpholin-4-yl-phenyl)-1.H,-pyrazolo[3,4-.b.]pyridin-6-yl]-amine;
(2,4-Difluoro-phenyl)-{3-[2-methoxy-5-(4-methyl-piperazin-1-yl)-phenyl]-1.H.-pyrazolo[3,4-.b.]pyridin-6-yl}-amine;
(2,4-Difluoro-phenyl)-[3-(2-methoxy-5-pyridin-4-yl-phenyl)-1.H.-pyrazolo[3,4-.b.]pyridin-6-yl]-amine;
(2,4-Difluoro-phenyl)-[3-(2-methoxy-5-methylaminomethyl-phenyl)-1.H.-pyrazolo[3,4-.b.]pyridin-6-yl]-amine;
(2,4-Difluoro-phenyl)-{3-[2-methoxy-5-(4-methyl-piperazin-1-ylmethyl)-phenyl]-1.H.-pyrazolo[3,4-.b.]pyridin-6-yl}-amine;
4-{3-[6-(2,4-Difluoro-phenylamino)-1H.-pyrazolo[3,4-.b.]pyridin-3-yl]-4-methoxy-phenyl}-1-methyl-piperidin-4-ol:
(2,4-Difluoro-phenyl)-[3-(2-methoxy-5-morpholin-4-ylmethyl-phenyl)-1.H.,pyrazolo[3,4-.b.]pyridin-6-yl]-amine;
(2,4-Difluoro-phenyl)-[3-(2-methoxy-5-piperazin-1-ylmethyl-phenyl)-1.H.-pyrazolo[3,4-.b.]pyridin-6-yl]-amine;
{3-[5-(3-Amino-3-methyl-but-1-ynyl)-2-methoxy-phenyl]-1.H.-pyrazolo[3,4-.b.]pyridin-6-yl}-(2,4-difluoro-phenyl)-amine;
{3-[5-(3-Amino-3-methyl-butyl)-2-methoxy-phenyl]-1.H.-pyrazolo[3,4-.b.]pyridin-6-yl}-(2,4-difluoro-phenyl)-amine;
{3-[2-Chloro-5-methoxy-4-(2-morpholin-4-yl-ethoxy).phenyl]-1.H-pyrazolo[3,4-.b.]pyridin-6-yl}-(2,4-difluoro-phenyl)-amine;
{3-[2-Chloro-4-methoxy-5-(2-morpholin-4-yl-ethoxy)-phenyl]-1.H-pyrazolo[3,4-.b.]pyridin-6-yl}-(2,4-difluoro-phenyl)-amine;
(2.4-Difluoro-phenyl)-[3-(2-methoxy-5-propylaminomethyl-phenyl)-1.H.-pyrazolo[3,4-.b.]pyridin-6-yl]-amine;
(2,4-Difluoro-phenyl)-(3-{2-methoxy-5-[(tetrahydro-pyran-4-ylamino)-methyl]-phenyl}-1.H.-pyrazolo[3,4-.b.]pyridin-6-yl)-amine;
(2,4-Difluoro-phenyl)-[3-[5-(1,2-dimethyl-1.H.-imidazol-4-yl)-2-methoxy-phenyl]-1.H.-pyrazolo[3,4-.b.]pyridin-6-yl}-amine;
3-[6-(2,4-Difluoro-phenylamino)-1.H.-pyrazolo[3,4-.b.]pyridin-3-yl]-4-methoxy-.N.-pyridin-2-yl-benzamide;
{3-[5-(3-Amino-3-methyl-but-1-ynyl)-2-methoxy-phenyl]-1.H.-pyrazolo[3,4-.d.]pyrimidin-6-yl}-(2,4-difluoro-phenyl)-amine;
4-{3-[6,(2,4-Difluoro-phenylamino)-1.H.-pyrazolo[3,4-.d.]pyrimidin-3-yl]-4-methoxy-phenyl}-2-methyl-but-3-yn-2-ol;
4-{3-[6-(2,4-Difluoro-phenylamino)-1.H.-pyrazolo[3.4-.d.]pyrimidin-3-yl]-4-methoxy-phenyl}-2-methyl-butan-2-ol;
(2,4-Difluoro-phenyl)-{3-[5-(3-dimethylamino-prop-1-ynyl)-2-methoxy-phenyl]-1.H.-pyrazolo[3,4-.d.]pyrimidin-6-y}-amine;
(2,4-Difluoro-phenyl)-{3-[2-methoxy-5-(3-morpholin-4-yl-prop-1-ynyl)-phenyl]-1.H.-pyrazolo[3.4-.d.]pyrimidin-6-yl}-amine;
(2,4-Difluoro-phenyl)-(3-{2-methoxy-5-[3-(4-methyl-piperazin-1-yl)-prop-1-ynyl]-phenyl}-1.H.-pyrazolo[3,4-.d.]pyrimidin-6-yl)-amine;
4-{3-[6-(2.4-Difluoro-phenylamino)-1.H.-pyrazolo[3,4-.d.]pyrimidin-3-yl]-4-methoxy-phenylethynyl}-1-methyl-piperidin-4-ol;
(2,4-Difluoro-phenyl)-{3-[5-((E)-3-dimethylamino-propenyl)-2-methoxy-phenyl]-1.H.-pyrazolo[3,4-.d.]pyrimidin-6-yl}-amine;
(2,4-Difluoro-phenyl)-3-[2-methoxy-5-((E)-3-morpholin-4-yl-propenyl)-phenyl]-1.H.-pyrazolo[3,4-.d.]pyrimidin-6-yl}-amine;
(2,4-Difluoro-phenyl)-(3-{2-methoxy-5-[(E)-3-(4-methyl-piperazin-1-yl)-propenyl]-phenyl}-1.H.-pyrazolo[3,4-.d.]pyrimidin-6-yl)-amine;
4-{3-[6-(2,4-Difluoro-phenylamino)-1.H,-pyrazolo[3,4-,b.]pyrazin-3-yl]-4-methoxy-phenyl}-2-methyl-but-3-yn-2-ol; and
(2,4-Difluoro-phenyl)-{3-[5-(3-dimethylamino-prop-1-ynyl)-2-methoxy-phenyl]-1.H.-pyrazolo[3,4-.b.]pyrazin-6-yl}-amine;
or a pharmaceutically-acceptable and -cleavable ester or acid addition salt thereof.

6. A compound according to any 1 of claims 1 to 5, a pharmaceutically acceptable and physiologically cleavable ester thereof or acid addition salt thereof, for use as a pharmaceutical, e.g. for use as an immunosuppressant or antiinflammatory agent.

7. A pharmaceutical composition comprising a compound according to any 1 of claims 1 to 5, a pharmaceutically acceptable and physiologically cleavable ester thereof or acid addition salt thereof, in association with a pharmaceutically acceptable diluent or carrier, e,g., for use as an immunosuppressant or anti-inflammatory agent.

8. Use of a compound according to any 1 of claims 1 to 5, a pharmaceutically acceptable and physiologically cleavable ester thereof or acid addition salt thereof in the manufacture of a medicament for use as an immunosuppressant or anti-inflammatory agent.

9. A process for the preparation of a compound of formula II 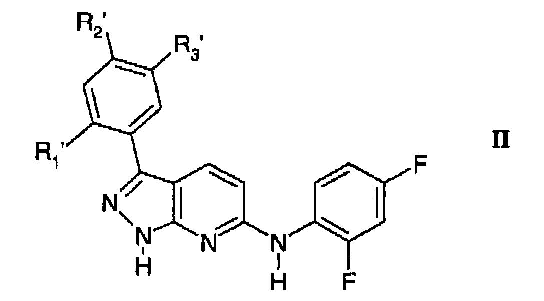 wherein R1', R2', and R3' are as defined in claim 3, comprising coupling a 6-chloro-3-phenyl-1.H.-pyrazolo[3,4-.b.]pyridine of formula III 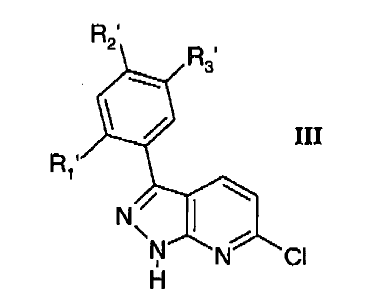 wherein R1', R2' and R3' are as defined above, with 2,4-difluoroaniline.

## Patentansprüche

1. Verbindung der Formel I 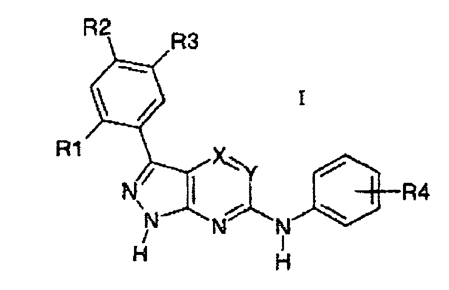 worin
X und Y unabhängig voneinander für Kohlenstoff oder Stickstoff stehen,
R1 für H, Halogen, Hydroxy, C₁-C₇-Alkoxy, C₁-C₇-Alkyl oder halogensubstituiertes C₁-C₇-Alkyl steht; R2 für H oder gegebenenfalls substituiertes (Heterocyclyl, C₁-C₇-Alkyl, C₂-C₇-Alkenyl, C₂-C₇-Alkinyl oder C₁-C₇-Alkoxy) steht;
R3 für H oder gegebenenfalls substituiertes (Heterocyclyl, C₁-C₇-Alkyl, C₂-C₇-Alkenyl, C₂-C₇-Alkinyl oder C₁-C₇-Alkoxy) steht; oder
R2 und R3 unter Bildung eines 4- bis 6-gliedrigen heterocyclischen Rings mit einem oder mehreren unter O, S, N oder NR, wobei R für H oder C₁-C₇-Alkyl steht, ausgewählten Heteroatomen miteinander verbunden sind;
R4 für einen, zwei oder drei Halogensubstituenten, die gleich oder verschieden sein können, steht, oder ein pharmazeutisch unbedenklicher und spaltbarer Ester oder ein pharmazeutisch unbedenkliches und spaltbares Säureadditionssalz davon,
mit Ausnahme von [3-(2-Chlorphenyl)-1H-pyrazolo[3,4-d]pyrimidin-6-yl](2-fluorphenyl)amin, [3-(2-Chlorphenyl)-1H-pyrazolo[3,4-b]pyrazin-6-yl] (2,4-difluorphenyl) amin und [3-(2-Chlorphenyl)-1H-pyrazolo[3,4-d]pyrimidin-6-yl](2,4-difluorphenyl)amin.

2. Verbindung nach Anspruch 1 der Formeln I', I" oder I"' 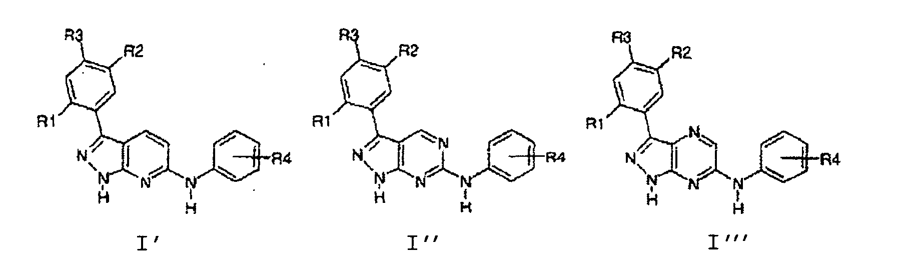 worin die Substituenten R1, R2, R3 und R4 die in Anspruch 1 angegebene Bedeutung besitzen,
oder ein pharmazeutisch unbedenklicher und spaltbarer Ester oder ein pharmazeutisch unbedenkliches und spaltbares Säureadditionssalz davon,
mit Ausnahme von [3-(2-Chlorphenyl)-1H-pyrazolo[3,4-d]pyrimidin-6-yl](2-fluorphenyl)amin, [3-(2-Chlorphenyl)-1H-pyrazolo[3,4-b]pyrazin-6-yl](2,4-difluorphenyl)amin und [3-(2-Chlorphenyl)-1H-pyrazolo [3,4-d] pyrimidin-6-yl] (2, 4-difluorphenyl)amin.

3. Verbindung der Formel I nach Anspruch 1, worin R4 für 2,4-Difluor steht und X, Y, R1, R2 und R3 die in Anspruch 1 angegebene Bedeutung besitzen, oder ein pharmazeutisch unbedenklicher und spaltbarer Ester oder ein pharmazeutisch unbedenkliches und spaltbares Säureadditionssalz davon, mit Ausnahme von [3-(2-Chlorphenyl)-1H-pyrazolo[3,4-b]pyrazin-6-yl](2,4-difluorphenyl)amin und [3-(2-Chlorphenyl)-1H-pyrazolo[3,4-d]pyrimidin-6-yl](2,4-difluorphenyl)amin.

4. Verbindung nach Anspruch 1 der Formel II 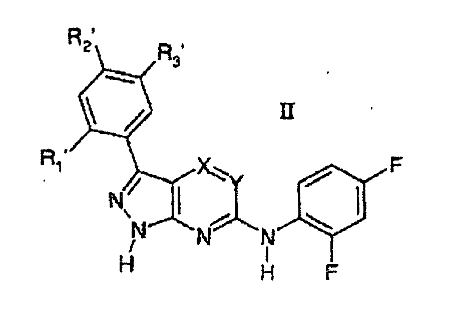 worin
X und Y unabhängig voneinander für Kohlenstoff oder Stickstoff stehen,
R1' für Halogen, C₁-C₄-Alkyl, C₁-C₄-Alkoxy oder halogensubstituiertes C₁-C₄-Alkyl steht;
R2' und R3' unabhängig voneinander für H oder gegebenenfalls substituiertes (C₁-C₄-Alkyl, C₁-C₄-Alkoxy, C₂-C₇-Alkenyl, C₂-C₇-Alkinyl, C₅-C₇-N-Heterocyclyl, C₅-C₇-Heterocyclyl-C₁-C₄-alkoxy, C₅-C₇-Heterocyclyl-C₁-C₄-alkyl, C₅-C₇-Heterocyclyl-C₂-C₄-alkenyl, C₅-C₇-Heterocyclyl-C₂-C₄-alkinyl) stehen, wobei das C₅-C₇-Heterocyclyl gegebenenfalls ein zweites Heteroatom enthält und die fakultative Substitution 1 oder 2 separat unter C₁-C₄-Alkyl, Halogen, Hydroxy, C₁-C₄-Alkoxy oder gegebenenfalls mono- oder di-N-C₁₋₄-alkyl-substituiertem Amino ausgewählte Substituenten umfaßt, oder
R2' und R3' über eine Methylendioxy-Brücke verbunden sind oder in einem gegebenenfalls durch 1 oder 2 separat unter C₁₋₄-Alkyl, Halogen, Hydroxy, C₁₋₄-Alkoxy und gegebenenfalls mono- oder di-N-C₁₋₄-alkyl-substituiertem Amino ausgewählte Substituenten substituierten Imidazolring verbunden sind; oder ein pharmazeutisch unbedenklicher und spaltbarer Ester oder ein pharmazeutisch unbedenkliches und spaltbares Säureadditionssalz davon,
mit Ausnahme von [3-(2-Chlorphenyl)-1H-pyrazolo[3,4-b]pyrazin-6-yl](2,4-difluorphenyl)-amin und [3-(2-Chlorphenyl)-1H-pyrazolo[3,4-d]-pyrimidin-6-yl](2,4-difluorphenyl)amin.

5. Verbindung der Formel I nach Anspruch 1, ausgewählt unter:
(2,4-Difluorphenyl)[3-(2-methoxyphenyl)-1H-pyrazolo[3,4-b]pyridin-6-yl]amin;
[3-(6-Chlorbenzo[1,3]dioxol-5-yl)-1H-pyrazolo[3,4-b]pyridin-6-yl](2,4-difluorphenyl)amin;
[3-(2-Chlorphenyl)-1H-pyrazolo[3,4-b]pyridin-6-yl](2,4-difluorphenyl)amin;
(2,4-Difluorphenyl)[3-(2-trifluormethylphenyl)-1H-pyrazolo[3,4-b]pyridin-6-yl]amin;
(2,4-Difluorphenyl)[3-(2,4-dimethoxyphenyl)-1H-pyrazolo[3,4-b]pyridin-6-yl]amin;
[3-(2-Chlor-4,5-dimethoxyphenyl)-1H-pyrazolo[3,4-b]pyridin-6-yl](2,4-difluorphenyl)amin;
(2,4-Difluorphenyl)[3-(2-methoxy-5-morpholin-4-ylphenyl)-1H-pyrazolo[3,4-b]pyridin-6-yl]amin;
(2,4-Difluorphenyl){3-[2-methoxy-5-(4-methyl-piperazin-1-yl)phenyl]-1H-pyrazolo[3,4-b]pyridin-6-yl}amin;
(2,4-Difluorphenyl)[3-(2-methoxy-5-pyridin-4-ylphenyl)-1H-pyrazolo[3,4-b]pyridin-6-yl]amin;
(2,4-Difluorphenyl)[3-(2-methoxy-5-methylamino-methylphenyl)-1H-pyrazolo[3,4-b]pyridin-6-yl]amin;
(2,4-Difluorphenyl){3-[2-methoxy-5-(4-methyl-piperazin-1-ylmethyl)phenyl]-1H-pyrazolo[3,4-b]pyridin-6-yl}amin;
4-{3-[6-(2,4-Difluorphenylamino)-1H-pyrazolo[3,4-b]pyridin-3-yl]-4-methoxyphenyl}-1-methylpiperidin-4-ol;
(2,4-Difluorphenyl)[3-(2-methoxy-5-morpholin-4-yl-methylphenyl)-1H-pyrazolo[3,4-b]pyridin-6-yl]amin;
(2,4-Difluorphenyl)[3-(2-methoxy-5-piperazin-1-yl-methylphenyl)-1H-pyrazolo[3,4-b]pyridin-6-yl]amin;
{3-[5-(3-Amino-3-methylbut-1-inyl)-2-methoxy-phenyl]-1H-pyrazolo[3,4-b]pyridin-6-yl}(2,4-difluorphenyl)amin;
{3-[5-(3-Amino-3-methylbutyl)-2-methoxyphenyl]-1H-pyrazolo[3,4-b]pyridin-6-yl}(2,4-difluorphenyl)amin;
{3-[2-Chlor-5-methoxy-4-(2-morpholin-4-ylethoxy)-phenyl]-1H-pyrazolo[3,4-b]pyridin-6-yl}(2,4-difluorphenyl)amin;
{3-[2-Chlor-4-methoxy-5-(2-morpholin-4-ylethoxy)-phenyl]-1H-pyrazolo[3,4-b]pyridin-6-yl}(2,4-difluorphenyl)amin;
(2,4-Difluorphenyl)[3-(2-methoxy-5-propylamino-methylphenyl)-1H-pyrazolo[3,4-b]pyridin-6-yl]amin;
(2,4-Difluorphenyl)(3-{2-methoxy-5-[(tetrahydropyran-4-ylamino)methyl]phenyl}-1H-pyrazolo[3,4-b]pyridin-6-yl]amin;
(2,4-Difluorphenyl){3-[5-(1,2-dimethyl-1H-imidazol-4-yl)-2-methoxyphenyl]-1H-pyrazolo[3,4-b]pyridin-6-yl}amin;
3-[6-(2,4-Difluorphenylamino)-1H-pyrazolo[3,4-b]pyridin-3-yl]-4-methoxy-N-pyridin-2-ylbenzamid;
{3-[5-(3-Amino-3-methylbut-1-inyl)-2-methoxy-phenyl]-1H-pyrazolo[3,4-d]pyrimidin-6-yl}(2,4-difluorphenyl)amin;
4-{3-[6-(2,4-Difluorphenylamino)-1H-pyrazolo[3,4-d]pyrimidin-3-yl]-4-methoxyphenyl}-2-methylbut-3-in-2-ol;
4-{3-[6-(2,4-Difluorphenylamino)-1H-pyrazolo[3,4-d]pyrimidin-3-yl]-4-methoxyphenyl}-2-methylbutan-2-ol;
(2,4-Difluorphenyl){3-[5-(3-dimethylaminoprop-1-inyl)-2-methoxyphenyl]-1H-pyrazolo[3,4-d]pyrimidin-6-yl}amin;
(2,4-Difluorphenyl){3-[2-methoxy-5-(3-morpholin-4-ylprop-1-inyl)phenyl]-1H-pyrazolo[3,4-d]pyrimidin-6-yl}amin;
(2,4-Difluorphenyl)(3-{2-methoxy-5-[3-(4-methyl-piperazin-1-yl)prop-1-inyl]phenyl}-1H-pyrazolo[3,4-d]pyrimidin-6-yl)amin;
4-{3-[6-(2,4-Difluorphenylamino)-1H-pyrazolo[3,4-d]pyrimidin-3-yl]-4-methoxyphenylethinyl}-1-methylpiperidin-4-ol;
(2,4-Difluorphenyl){3-[5-((E)-3-dimethylamino-propenyl)-2-methoxyphenyl]-1H-pyrazolo[3,4-d]-pyrimidin-6-yl}amin;
(2,4-Difluorphenyl){3-[2-methoxy-5-((E)-3-morpholin-4-ylpropenyl)phenyl]-1H-pyrazolo[3,4-d]-pyrimidin-6-yl}amin;
(2,4-Difluorphenyl)(3-{2-methoxy-5-[(E)-3-(4-methylpiperazin-1-yl)propenyl]phenyl}-1H-pyrazolo[3,4-d]pyrimidin-6-yl)amin;
4-{3-[6-(2,4-Difluorphenylamino)-1H-pyrazolo[3,4-b]pyrazin-3-yl]-4-methoxyphenyl}-2-methylbut-3-in-2-ol; und
(2,4-Difluorphenyl){3-[5-(3-dimethylaminoprop-1-inyl)-2-methoxyphenyl]-1H-pyrazolo[3,4-b]pyrazin-6-yl}amin;
oder ein pharmazeutisch unbedenklicher und spaltbarer Ester oder ein pharmazeutisch unbedenkliches und spaltbares Säureadditionssalz davon.

6. Verbindung nach einem der Ansprüche 1 bis 5, ein pharmazeutisch unbedenklicher und physiologisch spaltbarer Ester davon oder ein pharmazeutisch unbedenkliches und physiologisch spaltbares Säureadditionssalz davon zur Verwendung als Pharmazeutikum, z.B. zur Verwendung als Immunsuppressivum oder Antiphlogistikum.

7. Pharmazeutische Zusammensetzung, enthaltend eine Verbindung nach einem der Ansprüche 1 bis 5, einen pharmazeutisch unbedenklichen und physiologisch spaltbaren Ester davon oder ein pharmazeutisch unbedenkliches und physiologisch spaltbares Säureadditionssalz davon zusammen mit einem pharmazeutisch unbedenklichen Verdünnungsmittel oder Träger, z.B. zur Verwendung als Immunsuppressivum oder Antiphlogistikum.

8. Verwendung einer Verbindung nach einem der Ansprüche 1 bis 5, eines pharmazeutisch unbedenklichen und physiologisch spaltbaren Esters davon oder Säureadditionssalzes davon bei der Herstellung eines Arzneimittels zur Verwendung als Immunsuppressivum oder Antiphlogistikum.

9. Verfahren zur Herstellung einer Verbindung der Formel II 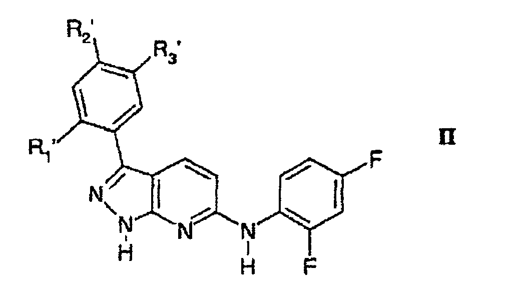 worin R1', R2' und R3' die in Anspruch 3 angegebene Bedeutung besitzen, bei dem man ein 6-Chlor-3-phenyl-1H-pyrazolo[3,4-b]pyridin der Formel III 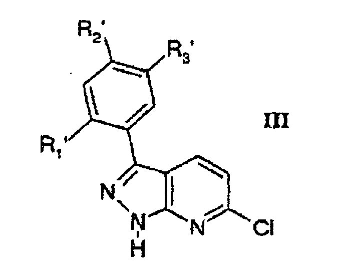 worin R1', R2' und R3' die oben angegebene Bedeutung besitzen, mit 2,4-Difluoranilin kuppelt.

## Revendications

1. Composé représenté par la formule I 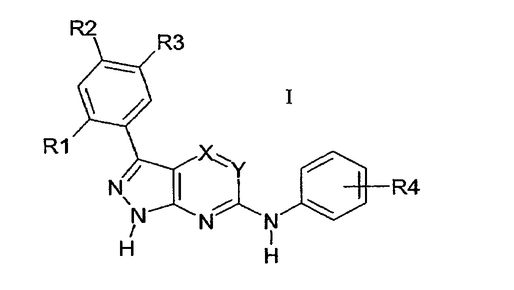 dans laquelle
X et Y sont indépendamment le carbone ou l'azote ;
R1 est H, un halogène, un groupe hydroxy, alcoxy en C₁-C₇, alkyle en C₁-C₇ ou alkyle en C₁-C₇ substitué par halogène ;
R2 est H, ou un groupe hétérocyclyle, alkyle en C₁-C₇, alcényle en C₂-C₇, alcynyle en C₂-C₇ ou alcoxy en C₁-C₇ facultativement substitué ;
R3 est H, ou un groupe hétérocyclyle, alkyle en C₁-C₇, alcényle en C₂-C₇, alcynyle en C₂-C₇ ou alcoxy en C₁-C₇ facultativement substitué ; ou
R2 et R3 sont liés ensemble pour former un cycle hétérocyclique de 4 à 6 chaînons contenant un ou plusieurs hétéroatomes choisis parmi O, S, N ou NR, où R est H ou un groupe alkyle en C₁-C₇ ;
R4 représente un, deux ou trois substituants halogènes qui peuvent être les mêmes ou différents ;
ou ester ou sel d'addition d'acide pharmaceutiquement acceptable et clivable de celui-ci, à l'exception de la [3-(2-chlorophényl)-*1H-*pyrazolo[3,4-d]pyrimidin-6-yl]-(2-fluorophényl)amine, la [3-{2-chlorophényl}-*1H*-pyrazolo[3,4-b]pyrazin-6-yl]-{2,4-difluorophényl)amine et la [3-(2-chlorophényl)-*1H-*pyrazolo[3,4-d]pyrimidin-6-yl]-(2,4-difluorophényl)amine.

2. Composé selon la revendication 1 représenté par les formules I', I" ou I"' 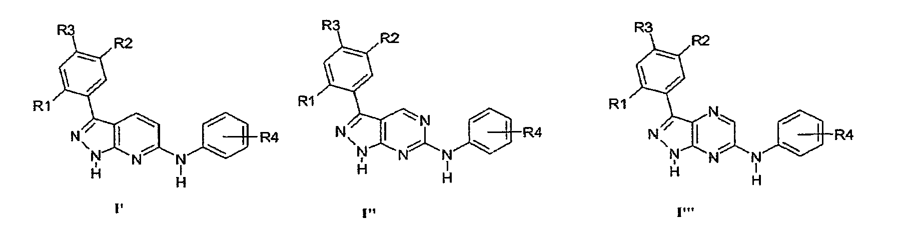 dans lesquelles les substituants R1, R2, R3 et R4 sont tels que définis dans la revendication 1,
ou ester ou sel d'addition d'acide pharmaceutiquement acceptable et clivable de celui-ci, à l'exception de la [3-(2-chlorophényl)-2H-pyrazolo[3,4-d]pyrimidin-6-yl]-(2-fluorophényl)amine,
la [3-(2-chlorophényl)-*1H*-pyrazolo[3,4-b]pyrazin-6-yl]-(2,4-difluorophényl)amine et la [3-(2-chlorophényl)-*1H-*pyrazolo[3,4-d]pyrimidin-6-yl]-(2,4-difluorophényl)amine.

3. Composé représenté par la formule I selon la revendication 1, dans lequel R4 est le substituant 2,4-difluoro et X, Y, R1, R2 et R3 sont tels que définis dans la revendication 1, ou ester, ou sel d'addition d'acide pharmaceutiquement acceptable et clivable de celui-ci, à l'exception de la [3-(2-chlorophényl)-1H-pyrazolo[3,4-b]pyrazin-6-yl]-(2,4-difluorophényl)amine et la [3-(2-chlorophényl)-*1H*-pyrazolo[3,4-d]pyrimidin-6-yl]-(2,4-difluorophényl)amine.

4. Composé selon la revendication 1 représenté par la formule II 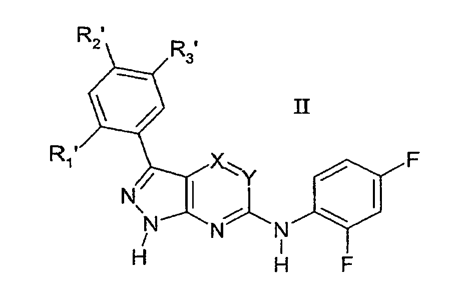 dans laquelle
X et Y sont indépendamment le carbone ou l'azote ;
R1' est un halogène, un groupe alkyle en C₁-C₄, alcoxy en C₁-C₄ ou alkyle en C₁-C₄ substitué par halogène ;
R2' et R3' sont chacun, indépendamment de l'autre, H ou un groupe alkyle en C₁-C₄, alcoxy en C₁-C₄, alcényle en C₂-C₇, alcynyle en C₂-C₇, N-hétérocyclyle en C₅-C₇, (hétérocyclyle en C₅-C₇) (alcoxy en C₁-C₄), (hétérocyclyle en C₅-C₇) (alkyle en C₁-C₄), (hétérocyclyle en C₅-C₇) (alcényle en C₂-C₄) ou (hétérocyclyle en C₅-C₇) (alcynyle en C₂-C₄) facultativement substitué, l'hétérocyclyle en C₅-C₇ contenant facultativement un second hétéroatome et la substitution facultative comprenant 1 ou 2 substituants, choisis séparément parmi un groupe alkyle en C₁-C₄, halogène, hydroxy, alcoxy en C₁-C₄ ou amino facultativement substitué par mono- ou di-*N*-(alkyle en C₁-C₄) ; ou
R2' et R3' sont liés par un groupe de liaison méthylènedioxy ou sont liés dans un cycle imidazole facultativement substitué par 1 ou 2 substituants, choisis séparément parmi un groupe alkyle en C₁-C₄, halogène, hydroxy, alcoxy en C₁-C₄ et amino facultativement substitué par mono- ou di-N-(alkyle en C₁-C₄) ;
ou ester ou sel d'addition d'acide pharmaceutiquement acceptable et clivable de celui-ci,
à l'exception de la [3-(2-chlorophényl)-*1H-*pyrazolo[3,4-b]pyrazin-6-yl]-(2,4-difluorophényl)amine et la [3-(2-chlorophényl)-*1H*-pyrazolo[3,4-d]pyrimidin-6-yl]-(2,4-difluorophényl)amine.

5. Composé représenté par la formule I selon la revendication 1 choisi parmi :
la (2,4-difluorophényl)-[3-(2-méthoxyphényl)-*1H-*pyrazolo[3,4-b]pyridin-6-yl]amine ;
la [3-(6-chlorobenzo[1,3]dioxol-5-yl)-*1H*-pyrazolo[3,4-b]pyridin-6-yl]-(2,4-difluorophényl)amine ;
la [3-(2-chlorophényl)-*1H*-pyrazolo[3,4-b]pyridin-6-yl]-(2,4-difluorophényl)amine ;
la (2,4-difluorophényl)-[3-(2-trifluorométhylphényl)-*1H*-pyrazolo[3,4-b]pyridin-6-yl]amine ;
la (2,4-difluorophényl)-[3-(2,4-diméthoxyphényl)-*1H-*pyrazolo[3,4-b]pyridin-6-yl]amine ;
la [3-(2-chloro-4,5-diméthoxyphényl)-*1H*-pyrazolo[3,4-b]pyridin-6-yl]-(2,4-difluorophényl)amine ;
la (2,4-difluorophényl)-[3-(2-méthoxy-5-morpholin-4-ylphényl)-*1H*-pyrazolo[3,4-b]pyridin-6-yl]amine ;
la (2,4-difluorophényl)-{3-[2-méthoxy-5-(4-méthylpipérazin-1-yl)phényl]-*1H*-pyrazolo[3,4-b]pyridin-6-yl}amine ;
la (2,4-difluorophényl)-[3-(2-méthoxy-5-pyridin-4-ylphényl)-*1H*-pyrazolo[3,4-b]pyridin-6-yl]amine ;
la (2,4-difluorophényl)-[3-(2-méthoxy-5-méthylaminométhylphényl)-*1H*-pyrazolo[3,4-b]pyridin-6-yl]amine ;
la (2,4-difluorophényl)-{3-[2-méthoxy-5-(4-méthylpipérazin-1-ylméthyl)phényl]-*1H*-pyrazolo[3,4-b]pyridin-6-yl}amine ;
le 4-{3-[6-(2,4-difluorophénylamino)-*1H*-pyrazolo[3,4-b]pyridin-3-yl]-4-méthoxyphényl}-1-méthylpipéridin-4-ol ;
la (2,4-difluorophényl)-[3-(2-méthoxy-5-morpholin-4-ylméthylphényl)-*1H*-pyrazolo[3,4-b]pyridin-6-yl]amine ;
la (2,4-difluorophényl)-[3-(2-méthoxy-5-pipérazin-1-ylméthylphényl)-*1H*-pyrazolo-[3,4-b]pyridin-6-yl]amine ;
la {3-[5-(3-amino-3-méthylbut-1-ynyl)-2-méthoxyphényl]-*1H*-pyrazolo[3,4-b]pyridin-6-yl}-(2,4-difluorophényl)amine ;
la {3-[5-(3-amino-3-méthylbutyl)-2-méthoxyphényl]-*1H-*pyrazolo[3,4-b]pyridin-6-yl}-(2,4-difluorophényl)amine ;
la {3-[2-chloro-5-méthoxy-4-(2-morpholin-4-yléthoxy)phényl]-*1H*-pyrazolo[3,4-b]pyridin-6-yl}-(2,4-difluorophényl)amine ;
la {3-[2-chloro-4-méthoxy-5-(2-morpholin-4-yléthoxy)phényl]-*1H*-pyrazolo[3,4-b]pyridin-6-yl}-(2,4-difluorophényl)amine ;
la (2,4-difluorophényl)-[3-(2-méthoxy-5-propylaminométhylphényl)-*1H*-pyrazolo[3,4-b]pyridin-6-yl]amine ;
la (2,4-difluorophényl)-(3-{2-méthoxy-5-[(tétrahydropyran-4-ylamino)méthyl]phényl}-*1H-*pyrazolo[3,4-b]pyridin-6-yl)amine ;
la (2,4-difluorophényl)-{3-[5-(1,2-diméthyl-*1H-*imidazol-4-yl)-2-méthoxyphényl]-*1H*-pyrazolo[3,4-b]pyridin-6-yl}amine ;
le 3-[6-(2,4-difluorophénylamino)-*1H*-pyrazolo[3,4-b]pyridin-3-yl]-4-méthoxy-N-pyridin-2-ylbenzamide ;
la {3-[5-(3-amino-3-méthylbut-1-ynyl)-2-méthoxyphényl]-*1H*-pyrazolo[3,4-d]pyrimidin-6-yl}-(2,4-difluorophényl)amine ;
le 4-{3-[6-(2,4-difluorophénylamino)-*1H*-pyrazolo[3,4-d]pyrimidin-3-yl]-4-méthoxyphényl}-2-méthylbut-3-yn-2-ol ;
le 4-{3-[6-(2,4-difluorophénylamino)-*1H*-pyrazolo[3,4-d]pyrimidin-3-yl]-4-méthoxyphényl}-2-méthylbutan-2-ol ;
la (2,4-difluorophényl)-{3-[5-(3-diméthylaminoprop-1-ynyl)-2-méthoxyphényl]-*1H*-pyrazolo[3,4-d]pyrimidin-6-yl}amine ;
la (2,4-difluorophényl)-{3-[2-méthoxy-5-(3-morpholin-4-yl-prop-1-ynyl)phényl]-*1H*-pyrazolo[3,4-d]pyrimidin-6-yl}amine ;
la (2,4-difluorophényl)-(3-{2-méthoxy-5-[3-(4-méthylpipérazin-1-yl)prop-1-ynyl]phényl}-*1H-*pyrazolo[3,4-d]pyrimidin-6-yl)amine ;
le 4-{3-[6-(2,4-difluorophénylamino)-*1H*-pyrazolo[3,4-d]pyrimidin-3-yl]-4-méthoxyphényléthynyl}-1-méthylpipéridin-4-ol ;
la (2,4-difluorophényl)-{3-[5-((*E*)-3-diméthylaminopropényl)2-méthoxyphényl]-*1H-*pyrazolo[3,4-d]pyrimidin-6-yl}amine ;
la (2,4-difluorophényl)-{3-[2-méthoxy-5-((*E*)-3-morpholin-4-yl-propényl)phényl]-*1H*-pyrazolo[3,4-d]pyrimidin-6-yl}amine ;
la (2,4-difluorophényl)-(3-{2-méthoxy-5-[(*E*)-3-(4-méthylpipérazin-1-yl)propényl]phényl}-*1H*-pyrazolo[3,4-d]pyrimidin-6-yl)amine ;
le 4-{3-[6-(2,4-difluorophénylamino)-*1H*-pyrazolo[3,4-b]pyrazin-3-yl]-4-méthoxyphényl}-2-méthylbut-3-yn-2-ol ; et
la (2,4-difluorophényl)-{3-[5-(3-diméthylaminoprop-1-ynyl)-2-méthoxyphényl]-*1H*-pyrazolo[3,4-b]pyrazin-6-yl}amine ;
ou ester ou sel d'addition d'acide pharmaceutiquement acceptable et clivable de celui-ci.

6. Composé selon l'une quelconque des revendications 1 à 5, ester de celui-ci ou sel d'addition d'acide de celui-ci pharmaceutiquement acceptable et physiologiquement clivable, destiné à être utilisé comme produit pharmaceutique, par exemple destiné à être utilisé comme agent immunosuppresseur ou anti-inflammatoire.

7. Composition pharmaceutique comprenant un composé selon l'une quelconque des revendications 1 à 5, un ester de celui-ci ou sel d'addition d'acide de celui-ci pharmaceutiquement acceptable et physiologiquement clivable, en association avec un diluant ou véhicule pharmaceutiquement acceptable, par exemple destinée à être utilisée comme agent immunosuppresseur ou anti-inflammatoire.

8. Utilisation d'un composé selon l'une quelconque des revendications 1 à 5, d'un ester de celui-ci ou sel d'addition d'acide de celui-ci pharmaceutiquement acceptable et physiologiquement clivable dans la fabrication d'un médicament destiné à être utilisé comme agent immunosuppresseur ou anti-inflammatoire.

9. Procédé pour la préparation d'un composé représenté par la formule II 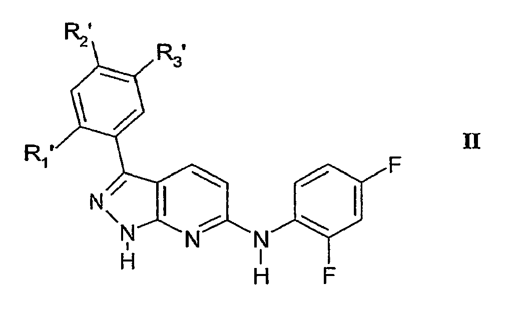 dans laquelle R1', R2' et R3' sont tels que définis dans la revendication 3, comprenant le couplage d'une 6-chloro-3-phényl-*1H*-pyrazolo[3,4-b]pyridine représentée par la formule III 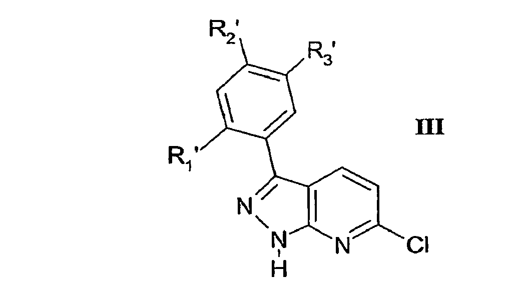 dans laquelle R1', R2' et R3' sont tels que définis ci-dessus, avec de la 2,4-difluoroaniline.
